(19) Europäisches Patentamt
European Patent Office
Office européen des brevets

(11) EP 3 493 851 B1

(12) **EUROPEAN PATENT SPECIFICATION**

(45) Date of publication and mention of the grant of the patent:
**25.06.2025 Bulletin 2025/26**

(21) Application number: **17837395.7**

(22) Date of filing: **20.07.2017**

(51) International Patent Classification (IPC):
***A61K 49/00*** (2006.01) ***G01N 33/574*** (2006.01)
***C12Q 1/44*** (2006.01) ***C07D 407/04*** (2006.01)
***C07D 493/10*** (2006.01) ***C09B 11/22*** (2006.01)
***C09K 11/07*** (2006.01) ***G01N 33/58*** (2006.01)

(52) Cooperative Patent Classification (CPC):
**A61K 49/0052; A61K 49/0041; A61K 49/0043; A61K 49/0071; A61K 49/0076; C07D 493/10**

(86) International application number:
**PCT/US2017/043141**

(87) International publication number:
**WO 2018/026538 (08.02.2018 Gazette 2018/06)**

(54) **NOVEL SCAFFOLDS FOR INTRACELLULAR COMPOUND DELIVERY FOR THE DETECTION OF CANCER CELLS**

NEUARTIGE GERÜSTE ZUR INTRAZELLULÄREN WIRKSTOFFABGABE ZUR DETEKTION VON KREBSZELLEN

NOUVEAUX ÉCHAFAUDAGES POUR L'ADMINISTRATION INTRACELLULAIRE DE COMPOSÉS POUR LA DÉTECTION DE CELLULES CANCÉREUSES.

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:
**02.08.2016 US 201662370130 P**
**22.08.2016 US 201662378128 P**
**12.09.2016 US 201662393524 P**
**13.01.2017 PCT/US2017/013531**
**15.05.2017 US 201715595930**
**15.05.2017 US 201715595920**

(43) Date of publication of application:
**12.06.2019 Bulletin 2019/24**

(73) Proprietors:
- **ISI Life Sciences, Inc.**
  **Newport Beach, California 92660 (US)**
- **Indicator Systems International, Inc.**
  **California 92660 (US)**
- **Swiss, Gerald F.**
  **Newport Beach, California 92660 (US)**
- **Moriarty, Robert**
  **Newport Beach, California 92660 (US)**
- **Pariza, Richard**
  **Newport, California 92660 (US)**
- **White, David**
  **Newport, California 92660 (US)**
- **Fante, John**
  **Newport, California 92660 (US)**
- **Hessler, III, Theodore**
  **Newport, California 92660 (US)**
- **Keshishian, Craig**
  **Newport Beach, California 92660 (US)**

(72) Inventors:
- **SWISS, Gerald F.**
  **Rancho Santa Fe, California 92067 (US)**
- **PARIZA, Richard**
  **Newport Beach, California 92660 (US)**
- **WHITE, David**
  **Newport Beach, California 92660 (US)**
- **FANTE, John**
  **Santa Barbara, California 93103 (US)**
- **HESSLER III, Theordore**
  **Hamburg, New Jersey 07419 (US)**
- **KESHISHIAN, Craig**
  **Newport Beach, California 92660 (US)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



**(Cont. next page)**

(74) Representative: **Carpmaels & Ransford LLP**
**One Southampton Row**
**London WC1B 5HA (GB)**

(56) References cited:

| | |
|---|---|
| **WO-A1-2015/024576** | **WO-A1-2015/073678** |
| **WO-A1-2016/123244** | **WO-A2-2017/124016** |
| **CN-A- 106 928 238** | **KR-A- 20150 090 673** |
| **US-A- 3 806 592** | **US-A1- 2002 110 691** |
| **US-A1- 2004 136 910** | **US-A1- 2006 128 033** |
| **US-A1- 2006 128 033** | **US-A1- 2013 344 002** |
| **US-A1- 2014 171 635** | **US-A1- 2014 227 297** |


- **T KOMATSU ET AL: "Real-Time Measurements of Protein Dynamics Using Fluorescence Activation-Coupled Protein Labeling Method", JOURNAL OF THE AMERICAN CHEMICAL SOCIETY, vol. 133, no. 17, 2011, pages 6745 - 6751, XP055695589**
- **H E. MURREY ET AL: "Systematic Evaluation of Bioorthogonal Reactions in Live Cells with Clickable HaloTag Ligands: Implications for Intracellular Imaging", JOURNAL OF THE AMERICAN CHEMICAL SOCIETY, vol. 137, no. 35, 2015, US, pages 11461 - 11475, XP055386114**
- **Z CHEN ET AL: "Second-Generation Covalent TMP-Tag for Live Cell Imaging", JOURNAL OF THE AMERICAN CHEMICAL SOCIETY, vol. 134, no. 33, 2012, pages 13692 - 13699, XP055695597**
- **S S. GALLAGHER ET AL: "An In Vivo Covalent TMP-Tag Based on Proximity-Induced Reactivity", ACS CHEMICAL BIOLOGY, vol. 4, no. 7, 2009, pages 547 - 556, XP055167892**
- **GOOITZEN M VAN DAM ET AL: "Intraoperative tumor-specific fluorescence imaging in ovarian cancer by folate receptor-[alpha] targeting: first in-human results", NATURE MEDICINE, vol. 17, no. 10, 2011, New York, pages 1315 - 1319, XP055299051**
- **RYAN P. JUDY ET AL: "Quantification of tumor fluorescence during intraoperative optical cancer imaging", SCIENTIFIC REPORTS, vol. 5, no. 1, 2015, XP055639973**
- **Y. LU ET AL: "Preclinical pharmacokinetics, tissue distribution, and antitumor activity of a folate-hapten conjugate-targeted immunotherapy in hapten-immunized mice", MOLECULAR CANCER THERAPEUTICS, vol. 5, no. 12, 2006, pages 3258 - 3267, XP055060935**

## Description

### *CROSS-REFERENCE TO RELATED APPLICATIONS*

This application claims the priority benefit of United States provisional application nos. 62/370,130 filed on August 2, 2016, 62/378,128 filed on August 22, 2016, and 62/393,524 filed on September 12, 2016.

This application is also a continuation in part of United States application no. 15/595,930 filed on May 15, 2017, a continuation in part of United States application of 15/595,920 filed on May 15, 2017, and a continuation in part of international application no. PCT/US2017/13531 filed January 13, 2017.

### *1. Field of the Invention*

This invention is directed to folic acid and pteroic acid conjugates. The conjugates described herein comprise a targeting agent that targets cancer cells with specificity. The targeting agent is covalently bound to a masked imaging agent through a bond or via a linker. As such, the conjugate first targets and is then absorbed by cancer cells. Upon absorption, the masked imaging agent is unmasked intracellularly to permit the imaging agent to be capable of producing a detectible signal.

### *2. State of the Art*

The art has long recognized the value of using targeting agents bound to imaging agents that could image aberrant mammalian cells such as those found in solid mass tumors. One example of such conjugates is found in US Patent No. 8,043,603. However, in that case, the imaging agent is never masked and is fully capable of producing a signal whether found intracellularly or extracellularly.

Such prior art imaging agents require intravenous injection hours prior to solid mass tumor resection so that these agents can preferentially accumulate into the tumor. While such accumulation provides images of such tumors, the ability to detect remnant tumor cells remaining at the margins after surgery remains elusive as most of the imaging agent used resides in the bulk of the tumor. However, it is well known that remnant tumor cells are responsible, as least in part, for recurrence of the cancer. US2013/344002A1 discloses folate targeted enhanced tumour and folate receptor positive tissue optical imaging technology.

The use of masked imaging agents that can bind to cancer cells with specificity and rapidly produce a signal when absorbed by such cancer cells and become unmasked is a long felt need in the art and one that would significantly improve remnant cancer cell detection and subsequent removal.

### *3. Summary of the Invention.*

This invention provides for the conjugates of claims 1-4. Disclosed are compounds (conjugates), compositions and methods whereby the compounds comprise a targeting (or delivering) agent that targets cancer cells with specificity and is subsequently absorbed by these cells. The targeting agent is covalently bound to a masked fluorescent imaging agent optionally through a linker. Upon absorption of the conjugate, the masked fluorescent imaging agent is unmasked so that the imaging agent is capable of producing a detectible fluorescent signal. The conjugates are preferably included in a composition that is capable of being applied a cellular mass suspected of containing cancer cells such as the surface of the surgical field after resection of the tumor.

The data establishes that the conjugates of this invention readily target and then are absorbed by cancer cells whereupon demasking of the imaging agent rapidly occurs thereby allowing the imaging agent to generate a fluorescent signal. In contrast thereto, the conjugates that remain masked do not produce a detectible signal and thus allow the clinician to readily identify remnant cancer cells after surgical resection of the solid mass tumor.

Repesentative conjugates of this invention are provided in Tables 1 and 2 below. Such conjugates include salts, tautomers and/or solvates thereof.

TABLE 1 - Folic Acid/Imaging Agents

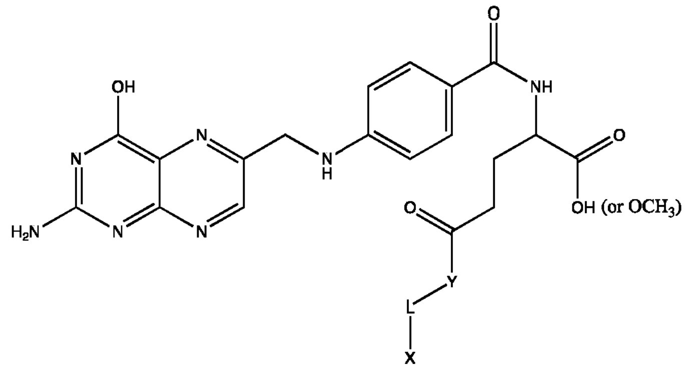

| | Y | L (from Y to X) | X | Imaging Agent |
|---|---|---|---|---|
| 1 | NH | $-(CH_2CH_2O)_3CH_2CH_2NH-CH_2C(O)NH-$ | 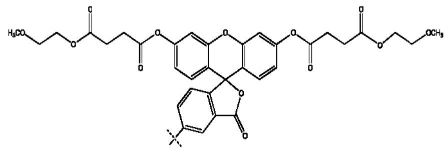 | di(methoxy-ethoxy) carbonylethyl carbonyloxy) 5-fluorescein |
| 2 | NH | $-(CH_2CH_2O)_3CH_2CH_2NH-C(S)NH-$ | 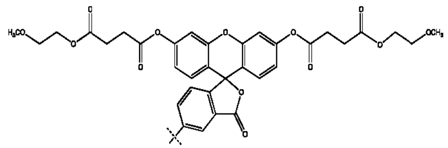 | di(methoxy-ethoxy) carbonylethyl carbonyloxy) fluorescein |
| 3 | NH | $-CH_2CH_2-$ | 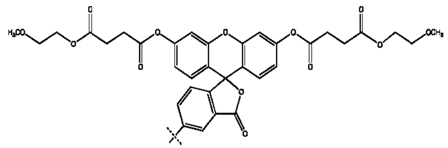 | di(methoxy-ethoxy) carbonylethyl carbonyloxy) 5-fluorescein |
| 4 | O | $-(CH_2CH_2O)_3CH_2CH_2O-C(S)NH-$ | 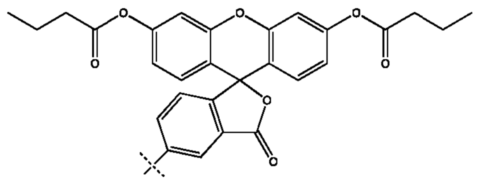 | di-(*n*-butyl-carbonyloxy) 5-fluores-cein |

(continued)

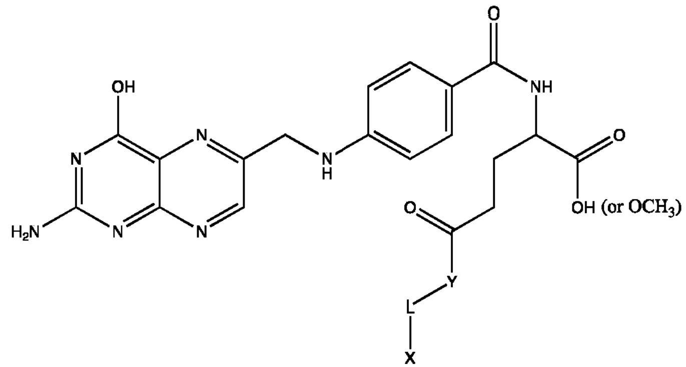

| | Y | L (from Y to X) | X | Imaging Agent |
|---|---|---|---|---|
| 5 | NH | $-(CH_2CH_2O)_3CH_2CH_2O-C(S)NH-$ | 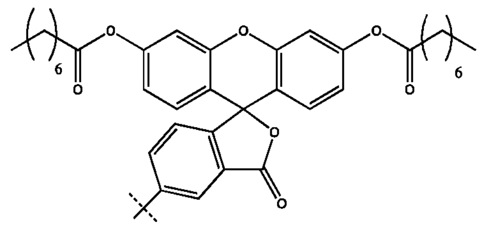 | di-(*n*-hexacarbonyloxy) 5-fluores-cein |
| 6 | NH | $-CH_2CH_2-$ | 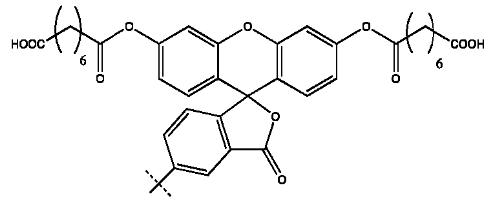 | di-(8-carboxy-hexacarbonyloxy) 5-fluorescein |
| 7 | NH | $-(CH_2CH_2O)_3CH_2CH_2NH-CH_2C(O)NH-$ | 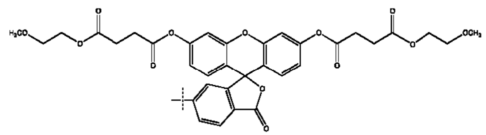 | di(methoxy-ethoxy) carbonylethyl carbonyloxy) 6-fluorescein |
| 8 | NH | $-(CH_2CH_2O)_3CH_2CH_2NH-C(S)NH-$ | 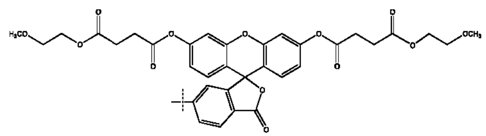 | di(methoxy-ethoxy) carbonylethyl carbonyloxy) 6-fluorescein |

(continued)

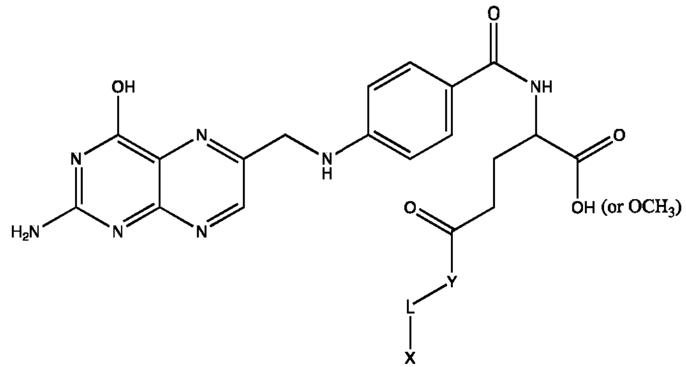

| | Y | L (from Y to X) | X | Imaging Agent |
|---|---|---|---|---|
| 9 | NH | $-CH_2CH_2-$ | 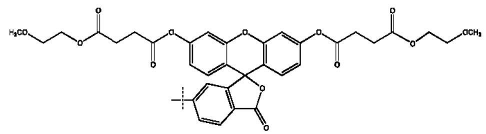 | di(methoxy-ethoxy) carbonylethyl carbonyloxy) 6-fluorescein |
| 10 | O | $-(CH_2CH_2O)_3CH_2CH_2O-C(S)NH-$ | 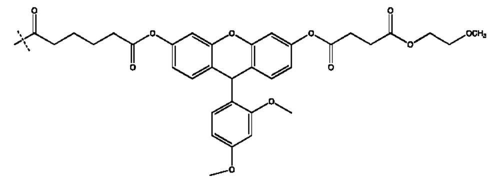 | |
| 11 | NH | $-(CH_2CH_2O)_3CH_2CH_2O-C(S)NH-$ | 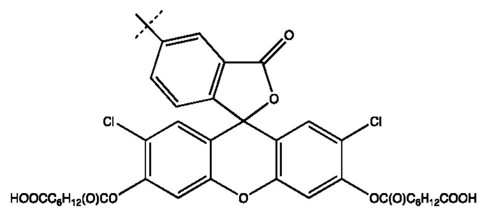 - | 5',7'-dichloro-di-(8-carboxy-hexa-carbonyloxy) 5-fluorescein |
| 12 | NH | $-(CH_2)_5-$ | 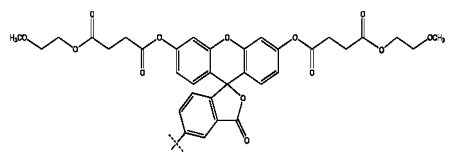 | di(methoxy-ethoxy) carbonylethyl carbonyloxy) 5-fluorescein |

TABLE 2 - Pteroic Acid/Imaging Agents

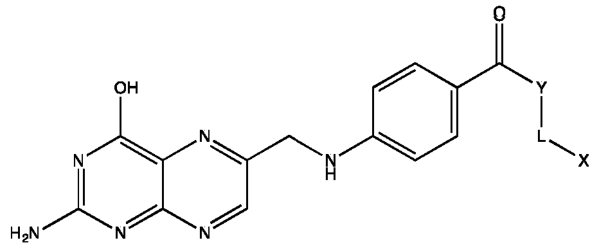

| | Y | L (from Y to X) | X | Imaging Agent |
|---|---|---|---|---|
| 13 | NH | $-(CH_2CH_2O)_3CH_2CH_2NH-CH_2C(O)NH-$ | 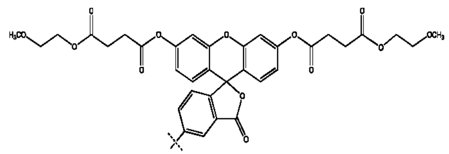 | di(methoxy-ethoxy) carbonylethyl carbonyloxy) 5-fluorescein |
| 14 | NH | $-(CH_2CH_2O)_3CH_2CH_2NH-C(S)NH-$ | 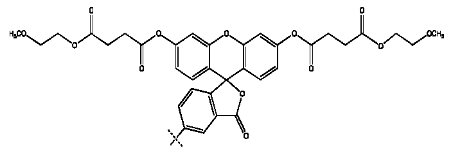 | di(methoxy-ethoxy) carbonylethyl carbonyloxy) fluorescein |
| 15 | NH | $-CH_2CH_2-$ | 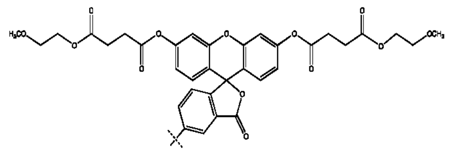 | di(methoxy-ethoxy) carbonylethyl carbonyloxy) 5-fluorescein |
| 16 | O | $-(CH_2CH_2O)_3CH_2CH_2O-C(S)NH-$ | 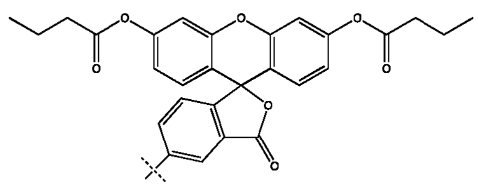 | di-(*n*-butyl-carbonyloxy) 5-fluorescein |

(continued)

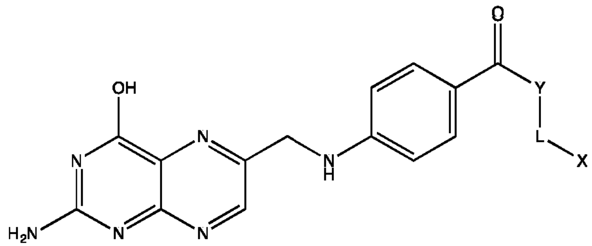

| | Y | L (from Y to X) | X | Imaging Agent |
|---|---|---|---|---|
| 17 | NH | $-(CH_2CH_2O)_3CH_2CH_2O-C(S)NH-$ | 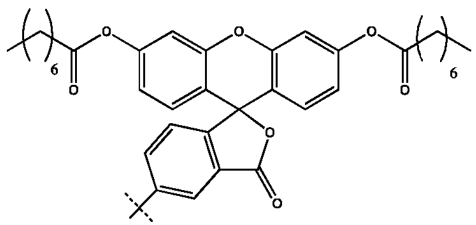 | di-(*n*-hexacarbonyloxy) 5-fluorescein |
| 18 | NH | $-CH_2CH_2-$ | 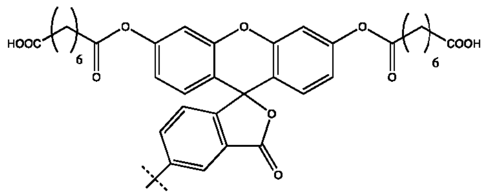 | di-(8-carboxy-hexacarbonyloxy) 5-fluor-escein |
| 19 | NH | $-(CH_2CH_2O)_3CH_2CH_2NH-CH_2C(O)NH-$ | 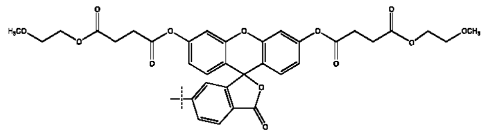 | di(methoxy-ethoxy) carbonylethyl carbonyloxy) 6-fluorescein |
| 20 | NH | $-(CH_2CH_2O)_3CH_2CH_2NH-C(S)NH-$ | 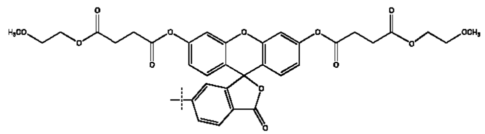 | di(methoxy-ethoxy) carbonylethyl carbo-nyloxy) 6-fluorescein |

(continued)

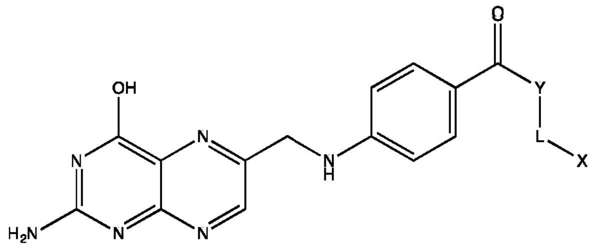

| | Y | L (from Y to X) | X | Imaging Agent |
|---|---|---|---|---|
| 21 | NH | $-CH_2CH_2-$ | 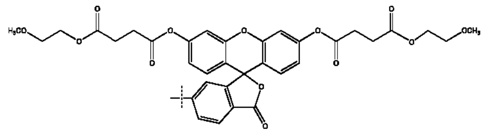 | di(methoxy-ethoxy) carbonylethyl carbonyloxy) 6-fluorescein |
| 22 | O | $-(CH_2CH_2O)_3CH_2CH_2O-C(S)NH-$ | 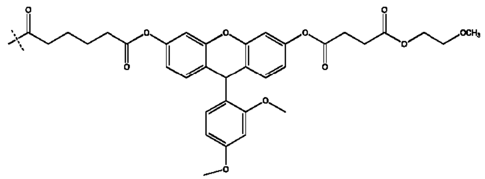 | |
| 23 | NH | $-(CH_2CH_2O)_3CH_2CH_2O-C(S)NH-$ | 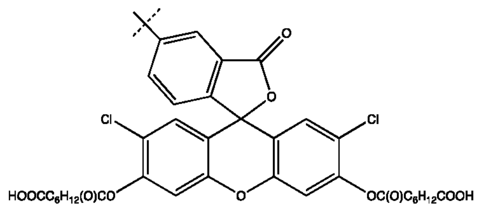 - | 5',7'-dichloro-di-(8-carboxy-hexacarbonyloxy) 5-fluorescein |

Further conjugates according to the invention including salts, solvates and tautomers thereof are provided in tables 3 and 4 below:

Table 3

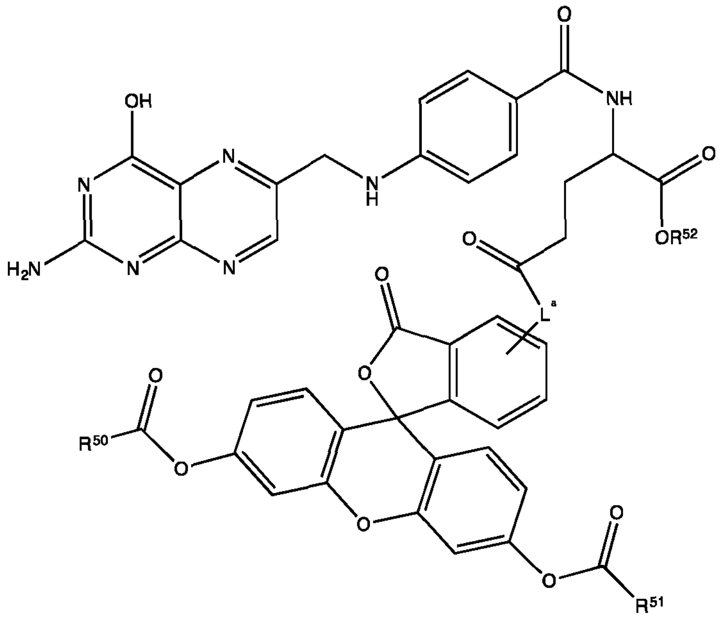

where

| $R^{50}/R^{51}$ | $R^{52}$ | $L^a$ |
|---|---|---|
| $CH_3OCH_2CH_2OC(O)CH_2CH_2C(O)O$-/ $CH_3OCH_2CH_2OC(O)CH_2CH_2C(O)O$- | H | 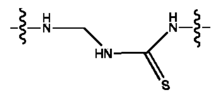 |
| $CH_3OCH_2CH_2OC(O)CH_2CH_2C(O)O$-/ $CH_3OCH_2CH_2OC(O)CH_2CH_2C(O)O$- | $CH_3$ | 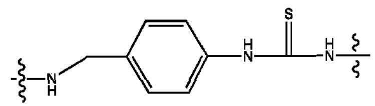 |
| $CH_3OCH_2CH_2OC(O)CH_2CH_2C(O)O$-/ $CH_3OCH_2CH_2OC(O)CH_2CH_2C(O)O$- | H | $-NHCH_2CH_2CH_2(OCH_2CH_2)_3CH_2NHC(=S)NH-$ |
| $CH_3OCH_2CH_2OC(O)CH_2CH_2C(O)O$-/ $CH_3OCH_2CH_2OC(O)CH_2CH_2C(O)O$- | H | 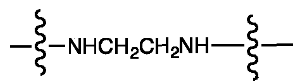 |
| $CH_3OCH_2CH_2OC(O)CH_2CH_2C(O)O-CH_3OCH_2CH_2OC(O)CH_2CH_2C(O)O$- | H | 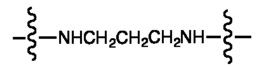 |

(continued)

| | | |
|---|---|---|
| 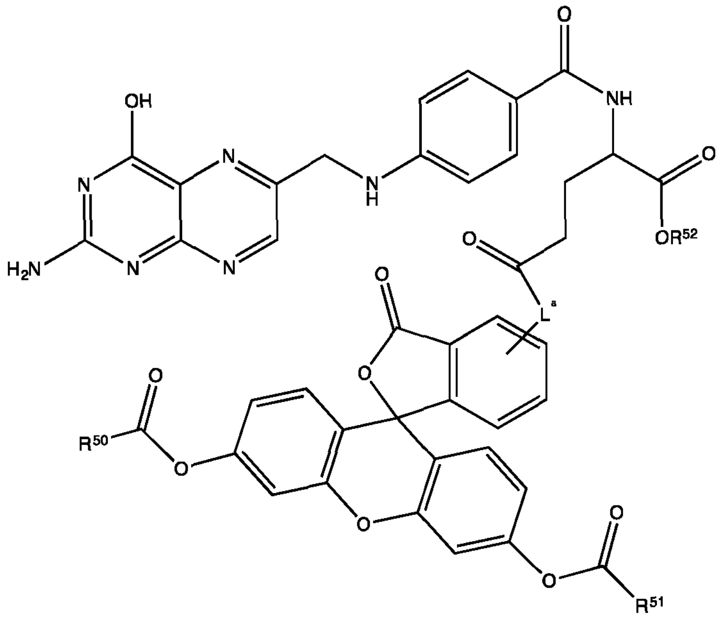<br>where | | |
| $CH_3OCH_2CH_2OC(O)CH_2CH_2C(O)O$-/ $CH_3CH_2C(O)O$- | H | 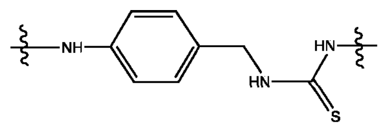 |
| $CH_3O_2C(CH_2CH_2O)_3C(O)(CH_2CH_2O)_3C(O)O$-$CH_3O_2C(CH_2CH_2O)_3C(O)$<br>$(CH_2CH_2O)_3C(O)O$- | H | 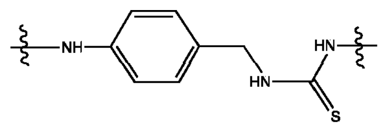 |
| $CH_3O(CH_2CH_2O)_2CH_2C(O)O$-/ $CH_3O(CH_2CH_2O)_2CH_2C(O)O$- | H | 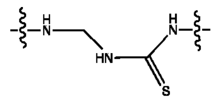 |

Table 4

| 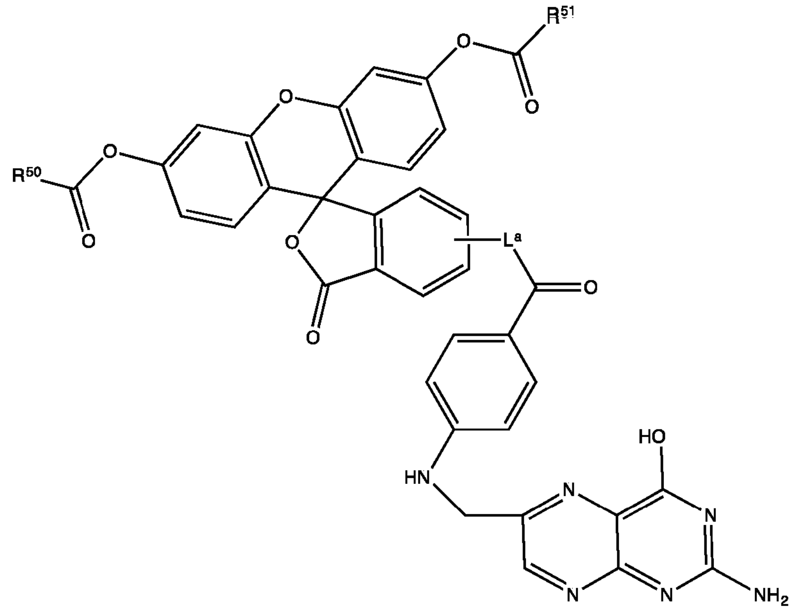 | |
|---|---|
| where | |
| $R^{50}/R^{51}$ | $L^a$ |
| $CH_3OCH_2CH_2OC(O)CH_2CH_2C(O)O$-/ $CH_3OCH_2CH_2OC(O)CH_2CH_2C(O)O$- | 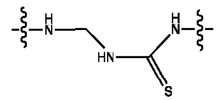 |
| $CH_3OCH_2CH_2OC(O)CH_2CH_2C(O)O$-/ $CH_3OCH_2CH_2OC(O)CH_2CH_2C(O)O$- | 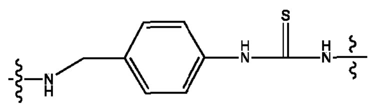 |
| $CH_3OCH_2CH_2OC(O)CH_2CH_2C(O)O$-/ $CH_3OCH_2CH_2OC(O)CH_2CH_2C(O)O$- | $-NHCH_2CH_2CH_2(OCH_2CH_2)_3CH_2NHC(=S)NH-$ |
| $CH_3OCH_2CH_2OC(O)CH_2CH_2C(O)O$-/ $CH_3OCH_2CH_2OC(O)CH_2CH_2C(O)O$- | 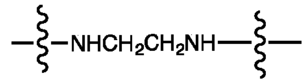 |

(continued)

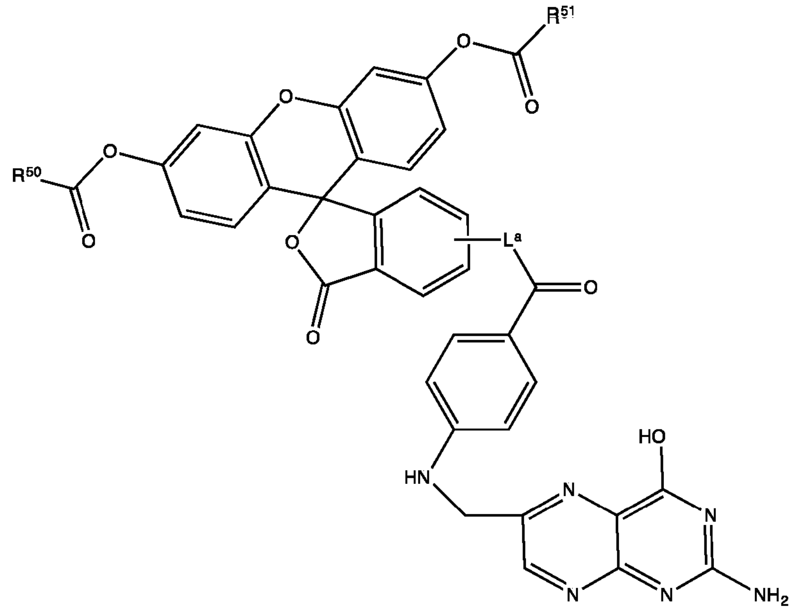

where

| $R^{50}/R^{51}$ | $L^a$ |
|---|---|
| $CH_3OCH_2CH_2OC(O)CH_2CH_2C(O)O$-$CH_3OCH_2CH_2OC(O)CH_2CH_2C(O)O$- | 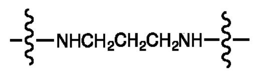 |
| $CH_3OCH_2CH_2OC(O)CH_2CH_2C(O)O$-$CH_3CH_2C(O)O$- | 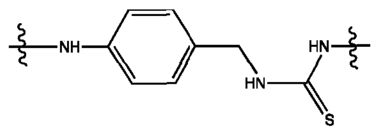 |
| $CH_3O_2C(CH_2CH_2O)_3C(O)(CH_2CH_2O)_3C(O)O$-$CH_3O_2C(CH_2CH_2O)_3C(O)(CH_2CH_2O)_3C(O)O$- | 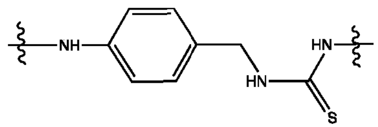 |

(continued)

| | |
|---|---|
| 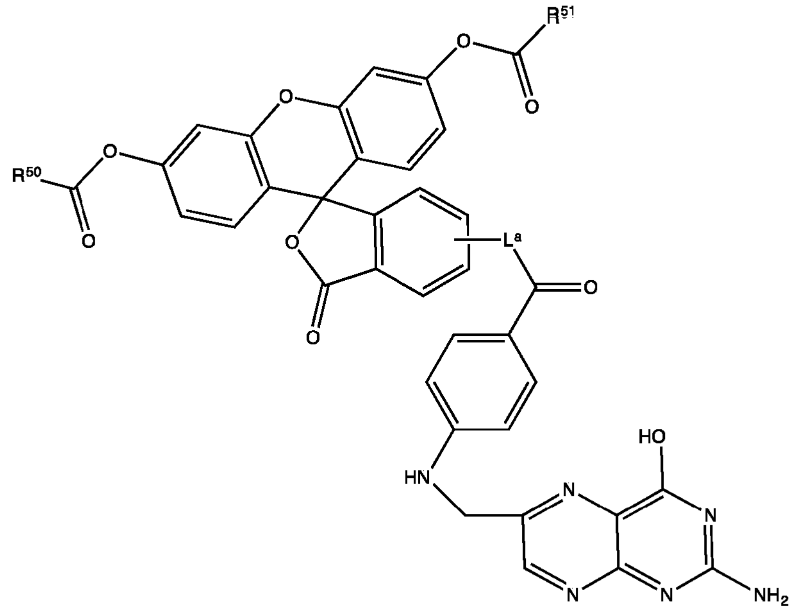 | |
| where | |
| $R^{50}/R^{51}$ | $L^a$ |
| $CH_3O(CH_2CH_2O)_2CH_2C(O)O$-/ $CH_3O(CH_2CH_2O)_2CH_2C(O)O$- | 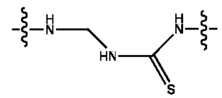 |

### *4. Brief Description of the Drawings*

FIG. 1 illustrates that an ovarian cancer cell line (SKOV3 cells) become fluorescent when exposed to the conjugates of this invention.

FIG. 2A and 2B illustrate that the mechanism by which the conjugates of this invention render these cancer cells fluorescent is via the folic acid receptor on these cells.

### *5. Detailed Description of the Invention*

After reading this description it will become apparent to one skilled in the art how to implement the invention in various alternative embodiments and alternative applications. It will be understood that the embodiments presented here are presented by way of an example only, and not limitation. As such, this detailed description of various alternative embodiments should not be construed to limit the scope or breadth of the present invention as set forth below.

Before the present invention is disclosed and described, it is to be understood that the aspects described below are not limited to specific compositions, methods of preparing such compositions, or uses thereof as such may, of course, vary. It is also to be understood that the terminology used herein is for the purpose of describing particular aspects only and is not intended to be limiting.

The specification is divided into various sections only for the reader's convenience and disclosure found in any section may be combined with that in another section. Titles or subtitles may be used in the specification for the convenience of a reader, which are not intended to influence the scope of the present invention.

### *5. Definitions*

Unless defined otherwise, all technical and scientific terms used herein have the same meaning as commonly understood by one of ordinary skill in the art to which this invention belongs. In this specification and in the claims that follow, reference will be made to a number of terms that shall be defined to have the following meanings:

The terminology used herein is for the purpose of describing particular embodiments only and is not intended to be limiting of the invention. As used herein, the singular forms "a", "an" and "the" are intended to include the plural forms as well, unless the context clearly indicates otherwise.

"Optional" or "optionally" means that the subsequently described event or circumstance can or cannot occur, and that the description includes instances where the event or circumstance occurs and instances where it does not.

The term "about" when used before a numerical designation, e.g., temperature, time, amount, concentration, and such other, including a range, indicates approximations which may vary by ( + ) or ( - ) 10%, 5%,1%, or any subrange or subvalue there between. Preferably, the term "about" when used with regard to a dose amount means that the dose may vary by +/- 10%.

"Comprising" or "comprises" is intended to mean that the compositions and methods include the recited elements, but not excluding others. "Consisting essentially of" when used to define compositions and methods, shall mean excluding other elements of any essential significance to the combination for the stated purpose. Thus, a composition consisting essentially of the elements as defined herein would not exclude other materials or steps that do not materially affect the basic and novel characteristic(s) of the disclosure. "Consisting of" shall mean excluding more than trace elements of other ingredients and substantial method steps.

"Alkyl" refers to monovalent saturated aliphatic hydrocarbyl groups having from 1 to 10 carbon atoms and preferably 1 to 6 carbon atoms. This term includes, by way of example, linear and branched hydrocarbyl groups such as methyl ($CH_3$-), ethyl ($CH_3CH_2$-), n-propyl ($CH_3CH_2CH_2$-), *iso*-propyl ($(CH_3)_2CH$-), n-butyl ($CH_3CH_2CH_2CH_2$-), iso-butyl ($(CH_3)_2CHCH_2$-), sec-butyl ($(CH_3)(CH_3CH_2)CH$-), t-butyl ($(CH_3)_3C$-), n-pentyl ($CH_3CH_2CH_2CH_2CH_2$-), and *neo*pentyl ($(CH_3)_3CCH_2$-).

"Heteroalkyl" refers to an alkyl group wherein 1-5 carbon atoms are replaced with one or more heteroatoms selected from >$NR^{30}$, -S-, -S(O)-, - $S(O)_2$-, and -O-, where $R^{30}$ is hydrogen, $C_1$-$C_6$ alkyl, or -C(O)$R^{31}$ is hydrogen or $C_1$-$C_6$ alkyl.

"Alkenyl" refers to monovalent straight or branched hydrocarbyl groups having from 2 to 10 carbon atoms and preferably 2 to 6 carbon atoms or preferably 2 to 4 carbon atoms and having at least 1 and preferably from 1 to 2 sites of vinyl (>C=C<) unsaturation. Such groups are exemplified, for example, by vinyl, allyl, and but-3-en-1-yl. Included within this term are the *cis* and *trans* isomers or mixtures of these isomers.

"Substituted alkyl" refers to an alkyl group having from 1 to 5, preferably 1 to 3, or more preferably 1 to 2 substituents selected from the group consisting of alkoxy, substituted alkoxy, acyl, acylamino, acyloxy, amino, substituted amino, aminocarbonyl, aminocarbonylamino, aminocarbonyloxy, aryl, substituted aryl, aryloxy, substituted aryloxy,

carboxyl, carboxyl ester, (carboxyl ester)amino, (carboxyl ester)oxy, cyano, cycloalkyl, substituted cycloalkyl, cycloalkyloxy, substituted cycloalkyloxy, halo, hydroxy, heteroaryl, substituted heteroaryl, heteroaryloxy, substituted heteroaryloxy, heterocyclic, substituted heterocyclic, heterocyclyloxy, substituted heterocyclyloxy, oxo and nitro.

"Substituted alkenyl" refers to alkenyl groups having from 1 to 5 substituents, and preferably 1 to 2 substituents, as defined above for substituted alkyl provided that any hydroxyl substitution is not attached to a vinyl (unsaturated) carbon atom.

"Alkylene" refers to divalent saturated aliphatic hydrocarbyl groups having from 1 to 10 carbon atoms, preferably having from 1 to 6 and more preferably 1 to 3 carbon atoms that are either straight-chained or branched. This term is exemplified by groups such as methylene ($-CH_2-$), ethylene ($-CH_2CH_2-$), *n*-propylene ($-CH_2CH_2CH_2-$), *iso*-propylene ($-CH_2CH(CH_3)-$ or $-CH(CH_3)CH_2-$), butylene ($-CH_2CH_2CH_2CH_2-$), *iso*-butylene ($-CH_2CH(CH_3)CH_2-$), *sec*-butylene ($-CH_2CH_2(CH_3)CH-$), and the like..

"Substituted alkylene" refers to an alkylene group having from from 1 to 5, preferably 1 to 3, or more preferably 1 to 2 substituents as defined above for substituted alkyl.

"Alkoxy" refers to the group -O-alkyl wherein alkyl is defined herein. Alkoxy includes, by way of example, methoxy, ethoxy, *n*-propoxy, *iso*-propoxy, *n*-butoxy, *t*-butoxy, *sec*-butoxy, and n-pentoxy.

"Substituted alkoxy" refers to the group -O-(substituted alkyl) wherein substituted alkyl is defined herein.

"Acyl" refers to the groups H-C(O)-, alkyl-C(O)-, substituted alkyl-C(O)-, alkenyl-C(O)-, substituted alkenyl-C(O)-, cycloalkyl-C(O)-, substituted cycloalkyl-C(O)-, cycloalkenyl-C(O)-, substituted cycloalkenyl-C(O)-, aryl-C(O)-, substituted aryl-C(O)-, heteroaryl-C(O)-, substituted heteroaryl-C(O)-, heterocyclic-C(O)-, and substituted heterocyclic-C(O)-. Acyl includes the "acetyl" group $CH_3C(O)-$.

"Acylamino" refers to the groups $-NR^{47}C(O)$alkyl, $-NR^{47}C(O)$substituted alkyl, $-NR^{47}C(O)$cycloalkyl, $-NR^{47}C(O)$ substituted cycloalkyl, $-NR^{47}C(O)$cycloalkenyl, $-NR^{47}C(O)$substituted cycloalkenyl, $-NR^{47}C(O)$alkenyl, $-NR^{47}C(O)$substituted alkenyl, $-NR^{47}C(O)$aryl, $-NR^{47}C(O)$substituted aryl, $-NR^{47}C(O)$heteroaryl, $-NR^{47}C(O)$substituted heteroaryl, $-NR^{47}C(O)$heterocyclic, and $-NR^{47}C(O)$substituted.

"Acyloxy" refers to the groups alkyl-C(O)O-, substituted alkyl-C(O)O-, alkenyl-C(O)O-, substituted alkenyl-C(O)O-, aryl-C(O)O-, substituted aryl-C(O)O-, cycloalkyl-C(O)O-, substituted cycloalkyl-C(O)O-, cycloalkenyl-C(O)O-, substituted cycloalkenyl-C(O)O-, heteroaryl-C(O)O-, substituted heteroaryl-C(O)O-, heterocyclic-C(O)O-, and substituted heterocyclic-C(O)O-.

"Amino" refers to the group $-NH_2$.

"Substituted amino" refers to the group $-NR^{48}R^{49}$ where $R^{48}$ and $R^{49}$ are independently selected from the group consisting of hydrogen, alkyl, substituted alkyl, alkenyl, substituted alkenyl, aryl, substituted aryl, cycloalkyl, substituted cycloalkyl, cycloalkenyl, substituted cycloalkenyl, heteroaryl, substituted heteroaryl, heterocyclic, and substituted heterocyclic, provided that $R^{48}$ and $R^{49}$ are both not hydrogen. When $R^{48}$ is hydrogen and $R^{49}$ is alkyl, the substituted amino group is sometimes referred to herein as alkylamino. When $R^{48}$ and $R^{49}$ are alkyl, the substituted amino group is sometimes referred to herein as dialkylamino. When referring to a monosubstituted amino, it is meant that either $R^{48}$ or $R^{49}$ is hydrogen but not both. When referring to a disubstituted amino, it is meant that neither $R^{48}$ nor $R^{49}$ are hydrogen.

"Aminocarbonyl" refers to the group $-C(O)NR^{50}R^{51}$ where $R^{50}$ and $R^{51}$ are independently selected from the group consisting of hydrogen, alkyl, substituted alkyl, alkenyl, substituted alkenyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where $R^{50}$ and $R^{51}$ are optionally joined together with the nitrogen bound thereto to form a heterocyclic or substituted heterocyclic group, and wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Aminothiocarbonyl" refers to the group $-C(S)NR^{50}R^{51}$ where $R^{50}$ and $R^{51}$ are independently selected from the group consisting of hydrogen, alkyl, substituted alkyl, alkenyl, substituted alkenyl, alkynyl, substituted alkynyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where $R^{50}$ and $R^{51}$ are optionally joined together with the nitrogen bound thereto to form a heterocyclic or substituted heterocyclic group.

"Aminocarbonylamino" refers to the group $-NR^{47}C(O)NR^{50}R^{51}$ where $R^{47}$ is hydrogen or alkyl and $R^{50}$ and $R^{51}$ are independently selected from the group consisting of hydrogen, alkyl, substituted alkyl, alkenyl, substituted alkenyl, aryl, substituted aryl, cycloalkyl, substituted cycloalkyl, cycloalkenyl, substituted cycloalkenyl, heteroaryl, substituted heteroaryl, heterocyclic, and substituted heterocyclic, and where $R^{50}$ and $R^{51}$ are optionally joined together with the nitrogen bound thereto to form a heterocyclic or substituted heterocyclic group.

"Aminocarbonyloxy" refers to the group $-O-C(O)NR^{50}R^{51}$ where $R^{50}$ and $R^{51}$ are independently selected from the group consisting of hydrogen, alkyl, substituted alkyl, alkenyl, substituted alkenyl, aryl, substituted aryl, cycloalkyl, substituted cycloalkyl, cycloalkenyl, substituted cycloalkenyl, heteroaryl, substituted heteroaryl, heterocyclic, and substituted heterocyclic and where $R^{50}$ and $R^{51}$ are optionally joined together with the nitrogen bound thereto to form a heterocyclic or substituted heterocyclic group.

"Aryl" or "Ar" refers to a monovalent aromatic carbocyclic group of from 6 to 14 carbon atoms having a single ring (*e.g.*, phenyl) or multiple condensed rings (*e.g.*, naphthyl or anthryl) which condensed rings may or may not be aromatic (*e. g.*, 2-benzoxazolinone, 2H-1,4-benzoxazin-3(4H)-one-7-yl, and the like) provided that the point of attachment is at an aromatic carbon atom. Preferred aryl groups include phenyl and naphthyl.

"Substituted aryl" refers to aryl groups which are substituted with 1 to 5, preferably 1 to 3, or more preferably 1 to 2 substituents selected from the group consisting of alkyl, substituted alkyl, alkenyl, substituted alkenyl, alkoxy, substituted alkoxy, acyl, acylamino, acyloxy, amino, substituted amino, aminocarbonyl, aminocarbonylamino, aminocarbonyloxy, aminosulfonyl, aminosulfonyloxy, aminosulfonylamino, aryl, substituted aryl, aryloxy, substituted aryloxy, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkenyl, substituted cycloalkenyl, cycloalkenyloxy, substituted cycloalkenyloxy, halo, hydroxy, heteroaryl, substituted heteroaryl, heteroaryloxy, substituted heteroaryloxy, heterocyclic, substituted heterocyclic, heterocyclyloxy, substituted heterocyclyloxy, and nitro.

"Arylene" refers to a divalent aromatic carbocyclic group of from 6 to 14 carbon atoms having a single ring or multiple condensed rings. "Substituted arylene" refers to an arylene having from 1 to 5, preferably 1 to 3, or more preferably 1 to 2 substituents as defined for aryl groups.

"Aryloxy" refers to the group -O-aryl, where aryl is as defined herein, that includes, by way of example, phenoxy and naphthoxy.

"Substituted aryloxy" refers to the group -O-(substituted aryl) where substituted aryl is as defined herein.

"Carbonyl" refers to the divalent group -C(O)- which is equivalent to -C(=O)-.

"Carboxyl" or "carboxy" refers to -COOH or salts thereof.

"Carboxyl ester" or "carboxy ester" refers to the group -C(O)(O)-alkyl, -C(O)(O)-substituted alkyl, -C(O)O-alkenyl, -C(O)(O)-substituted alkenyl, -C(O)(O)-aryl, -C(O)(O)-substituted-aryl, -C(O)(O)-cycloalkyl, -C(O)(O)-substituted cycloalkyl, -C(O)(O)-cycloalkenyl, -C(O)(O)-substituted cycloalkenyl, -C(O)(O)-heteroaryl, -C(O)(O)-substituted heteroaryl, - C(O)(O)-heterocyclic, and -C(O)(O)-substituted heterocyclic.

"(Carboxyl ester)amino refers to the group $-NR^{47}C(O)(O)$-alkyl, $-NR^{47}C(O)(O)$-substituted alkyl, $-NR^{47}C(O)O$-alkenyl, $-NR^{47}C(O)(O)$-substituted alkenyl, $-NR^{47}C(O)(O)$-aryl, $-NR^{47}C(O)(O)$-substituted-aryl, $-NR^{47}C(O)(O)$-cycloalkyl, $-NR^{47}C(O)(O)$-substituted cycloalkyl, $-NR^{47}C(O)(O)$-cycloalkenyl, - $NR^{47}C(O)(O)$-substituted cycloalkenyl, $-NR^{47}C(O)(O)$-heteroaryl, $-NR^{47}C(O)(O)$-substituted heteroaryl, $-NR^{47}C(O)(O)$-heterocyclic, and $-NR^{47}C(O)(O)$-substituted heterocyclic wherein $R^{47}$ is alkyl or hydrogen, and wherein alkyl, substituted alkyl, alkenyl, substituted alkenyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"(Carboxyl ester)oxy refers to the group -O-C(O)O-alkyl, -O-C(O)O-substituted alkyl, -O-C(O)O-alkenyl, -O-C(O)O-substituted alkenyl, -O-C(O)O-aryl, -O-C(O)O-substituted-aryl, -O-C(O)O-cycloalkyl, -O-C(O)O-substituted cycloalkyl, -O-C(O)O-cycloalkenyl, -O-C(O)O-substituted cycloalkenyl, -O-C(O)O-heteroaryl, -O-C(O)O-substituted heteroaryl, -O-C(O)O-heterocyclic, and -O-C(O)O-substituted heterocyclic wherein alkyl, substituted alkyl, alkenyl, substituted alkenyl, alkynyl, substituted alkynyl, cycloalkyl, substituted cycloalkyl, cycloalkenyl, substituted cycloalkenyl, aryl, substituted aryl, heteroaryl, substituted heteroaryl, heterocyclic, and substituted heterocyclic are as defined herein.

"Cyano" refers to the group -CN.

"Cycloalkyl" refers to cyclic alkyl groups of from 3 to 10 carbon atoms having single or multiple cyclic rings including fused, bridged, and spiro ring systems. The fused ring can be an aryl ring provided that the non aryl part is joined to the rest of the molecule. Examples of suitable cycloalkyl groups include, for instance, adamantyl, cyclopropyl, cyclobutyl, cyclopentyl, and cyclooctyl.

"Cycloalkenyl" refers to non-aromatic cyclic alkyl groups of from 3 to 10 carbon atoms having single or multiple cyclic rings and having at least one >C=C< ring unsaturation and preferably from 1 to 2 sites of >C=C< ring unsaturation.

"Substituted cycloalkyl" and "substituted cycloalkenyl" refers to a cycloalkyl or cycloalkenyl group having from 1 to 5 or preferably 1 to 3 substituents selected from the group consisting of oxo, thioxo, alkyl, substituted alkyl, alkenyl, substituted alkenyl, alkoxy, substituted alkoxy, acyl, acylamino, acyloxy, amino, substituted amino, aminocarbonyl, aminocarbonylamino, aminocarbonyloxy, aryl, substituted aryl, aryloxy, substituted aryloxy, carboxyl, carboxyl ester, (carboxyl ester)amino, (carboxyl ester)oxy, cyano, cycloalkyl, substituted cycloalkyl, cycloalkyloxy, substituted cycloalkyloxy, cycloalkenyl, substituted cycloalkenyl, cycloalkenyloxy, substituted cycloalkenyloxy, halo, hydroxy, heteroaryl, substituted heteroaryl, heteroaryloxy, substituted heteroaryloxy, heterocyclic, substituted heterocyclic, heterocyclyloxy, substituted heterocyclyloxy, and nitro.

"Cycloalkyloxy" refers to -O-cycloalkyl.

"Substituted cycloalkyloxy refers to -O-(substituted cycloalkyl).

"Cycloalkenyloxy" refers to -O-cycloalkenyl.

"Substituted cycloalkenyloxy" refers to -O-(substituted cycloalkenyl).

"Guanidino" refers to the group $-NHC(=NH)NH_2$.

"Halo" or "halogen" refers to fluoro, chloro, bromo and iodo.

"Hydroxy" or "hydroxyl" refers to the group -OH.

"Heteroaryl" refers to an aromatic group of from 1 to 10 carbon atoms and 1 to 4 heteroatoms selected from the group consisting of oxygen, nitrogen and sulfur within the ring. Such heteroaryl groups can have a single ring (*e.g.*, pyridinyl or furyl) or multiple condensed rings (*e.g.*, indolizinyl or benzothienyl) wherein the condensed rings may or may not be aromatic and/or contain a heteroatom provided that the point of attachment is through an atom of the aromatic heteroaryl group. In one embodiment, the nitrogen and/or the sulfur ring atom(s) of the heteroaryl group are optionally oxidized to provide for the N-oxide (N→O), sulfinyl, or sulfonyl moieties. Certain non-limiting examples include pyridinyl, pyrrolyl, indolyl, thiophenyl, oxazolyl, thizolyl, and furanyl.

"Substituted heteroaryl" refers to heteroaryl groups that are substituted with from 1 to 5, preferably 1 to 3, or more preferably 1 to 2 substituents selected from the group consisting of the same group of substituents defined for substituted aryl.

"Heteroaryloxy" refers to -O-heteroaryl.

"Substituted heteroaryloxy" refers to the group -O-(substituted heteroaryl).

"Heterocycle" or "heterocyclic" or "heterocycloalkyl" or "heterocyclyl" refers to a saturated or partially saturated, but not aromatic, group having from 1 to 10 ring carbon atoms and from 1 to 4 ring heteroatoms selected from the group consisting of nitrogen, sulfur, or oxygen. Heterocycle encompasses single ring or multiple condensed rings, including fused bridged and spiro ring systems. In fused ring systems, one or more the rings can be cycloalkyl, aryl, or heteroaryl provided that the point of attachment is through a non-aromatic ring. In one embodiment, the nitrogen and/or sulfur atom(s) of the heterocyclic group are optionally oxidized to provide for the N-oxide, sulfinyl, or sulfonyl moieties.

"Substituted heterocyclic" or "substituted heterocycloalkyl" or "substituted heterocyclyl" refers to heterocyclyl groups that are substituted with from 1 to 5 or preferably 1 to 3 of the same substituents as defined for substituted cycloalkyl.

"Heterocyclyloxy" refers to the group -O-heterocycyl.

"Substituted heterocyclyloxy" refers to the group -O-(substituted heterocycyl).

Examples of heterocycle and heteroaryls include, but are not limited to, azetidine, pyrrole, furan, thiophene, imidazole, pyrazole, pyridine, pyrazine, pyrimidine, pyridazine, indolizine, isoindole, indole, dihydroindole, indazole, purine, quinolizine, isoquinoline, quinoline, phthalazine, naphthylpyridine, quinoxaline, quinazoline, cinnoline, pteridine, carbazole, carboline, phenanthridine, acridine, phenanthroline, isothiazole, phenazine, isoxazole, phenoxazine, phenothiazine, imidazolidine, imidazoline, piperidine, piperazine, indoline, phthalimide, 1,2,3,4-tetrahydroisoquinoline, 4,5,6,7-tetrahydrobenzo[b]thiophene, thiazole, thiazolidine, thiophene, benzo[b]thiophene, morpholinyl, thiomorpholinyl (also referred to as thiamorpholinyl), 1,1-dioxothiomorpholinyl, piperidinyl, pyrrolidine, and tetrahydrofuranyl.

"Nitro" refers to the group $-NO_2$.

"Oxo" refers to the atom (=O).

The term "oxyalkylene" refers to -O-alkylene- and the term "polyoxyalkylene" refers to "$-(O\text{-alkylene})_n$ where *n* is an integer from 1 to 15.

Unless indicated otherwise, the nomenclature of substituents that are not defined herein are arrived at by naming the terminal portion of the functionality followed by the adjacent functionality toward the point of attachment. For example, the substituent "alkoxycarbonylalkyl" refers to (alkoxy)-C(O)-(alkyl)-. Likewise, "alkarylene" refers to -alkylene-arylene- and "arylalkylene" refers to -arylene-alkylene-.

It is understood that in all substituted groups defined above, polymers arrived at by defining substituents with further substituents to themselves (e.g., substituted aryl having a substituted aryl group as a substituent which is itself substituted with a substituted aryl group, etc.) are not intended for inclusion herein. In such cases, the maximum number of such substituents is three. That is to say that each of the above definitions is constrained by a limitation that, for example, substituted aryl groups are limited to -substituted aryl-(substituted aryl)-substituted aryl.

It is understood that the above definitions are not intended to include impermissible substitution patterns (e.g., methyl substituted with 5 fluoro groups). Such impermissible substitution patterns are well known to the skilled artisan.

"Tautomer" refer to alternate forms of a compound that differ in the position of a proton, such as enol-keto and imine-enamine tautomers, or the tautomeric forms of heteroaryl groups containing a ring atom attached to both a ring -NH- moiety and a ring =N- moiety such as pyrazoles, imidazoles, benzimidazoles, triazoles, and tetrazoles. Fluorescein is an example of a compound with tautomeric structures as depicted below:

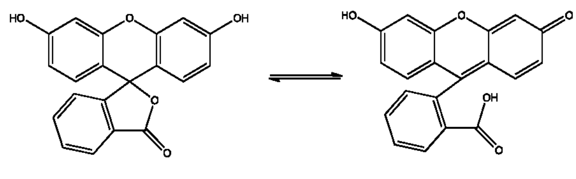

Likewise, folic acid (as well as pteroic acid) is another example of a compound with tautomeric structures as depicted

below:

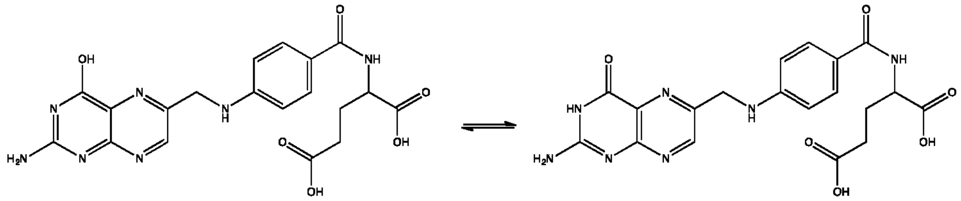

It is understood that depiction of any tautomer structure herein is intended to include their corresponding tautomers.

As used herein, the term stereo chemically pure denotes a compound which has 80% or greater by weight of the indicated stereoisomer and 20% or less by weight of other stereoisomers. In a further embodiment, the compounds of this invention have 90% or greater by weight of the stated stereoisomer and 10% or less by weight of other stereoisomers. In a yet further embodiment, the compounds of this invention have 95% or greater by weight of the stated stereoisomer and 5% or less by weight of other stereoisomers. In a still further embodiment, the compounds of this invention have 97% or greater by weight of the stated stereoisomer and 3% or less by weight of other stereoisomers.

The term "fluorescent masking agent" refers to a group covalently bound to a fluorescent moiety through a cleavable bond that significantly limits or eliminates the ability of that fluorescent moiety to fluoresce. That is to say that the signal generating capacity of the fluorescent moiety has been reduced by at least 90% or more and preferably completely eliminated. When so modified, the masked fluorescent compound is sometimes referred to herein as a "pro-fluorescent compound". Examples of such masking include acylation of both hydroxyl groups of fluorescein and derivatives thereof. When so acylated, the fluorescein and its derivatives are locked into a structure that cannot fluoresce. Such masking agents do not include quenchers whether associated with or bound to a fluorophore as the underlying fluorophore still generates a fluorescent signal but that signal is captured by the quencher as opposed to masking the fluorophore by reducing or eliminating its fluorescence.

The term "unmasked" refers to removal of the masking agent(s) from the masked fluorescent imaging agent so that the imaging agent can once again generate fluorescence. Unmasking generally requires cleavage of a cleavable bond that couples the masking agent to the conjugate and, preferably, to the imaging agent.

The term "cleavable bond" refers to bonds that stable in the extracellular domain but are cleaved in the intracellular domain of cancer cells. Such cleavable bonds include glycosides, esters, orthoesters, carbonates, and the like. Esters are a particularly preferred cleavable bond.

The term "stable" refers to a bond that has a sufficient half-life in physiological fluids including serum such that there is preferential targeting of the pro-fluorescent moiety to the cancer cell. One preferred half-life is at least about 2 minutes, more preferably at least 5 minutes and even more preferably at least 15 minutes.

The term "targeting agent" refers to any component or moiety that binds with specificity to a cancer cell through a receptor that permits absorption of such targeting agents into the cell. For the purposes of this application, such targeting agents do not include antibodies such as monoclonal antibodies as absorption into such cell does not typically occur with antibodies. Examples of targeting agents include folic acid, pteroic acid, folic acid alpha $C^1$-$C_4$ alkyl ester, luteinizing hormone releasing hormone (LHRH), Dharap, et al., PNAS, 120(36):12962-12967 (2005), avidin/streptavidin (binds to the D-galactose receptors commonly found on cancer cells, see e.g. Ogawa et al. Mol. Pharm. 2009; 6(2): 386-395), and the like.

The term "fluorescent imaging agent" refers to imaging agents that produce a fluorescent signal upon exposure to ultraviolet light and which agents are capable of being masked by covalent linkage of a moiety or moieties thereto provided that such moiety or moieties do not constitute quenchers. For the sake of completion, a "quencher" is a material that is associated with or bound to a fluorescent compound or moiety that, because of its proximity to the fluorescent compound or moiety, absorbs the fluorescence generated by that compound or moiety. Such is in contrast to a masking agent that prevents or reduces the fluorescence generated by the fluorescent compound or moiety.

The term "conjugate" or "compound" refers to a molecule comprising a targeting agent and a fluorescent imaging agent as components (or moieties) of that compound or conjugate. The fluorescent imaging agent is bound to the targeting agent via a covalent bond. In one embodiment, the fluorescent imaging agent is masked by ester group(s) that can readily be cleaved under intracellular conditions.

The term "cleaved" refers to breakage of the cleavable bond thereby regenerating the signal generating capacity of the previously pro-fluorescent moiety.

The term "linker" refers to a linking group of from 1 to 30 carbon atoms in length optionally having from 1 to 15 heteroatoms such as oxygen, oxo (=O), thioxo (=S), phosphorus (including oxides thereof such as phosphite, phosphate, etc.), sulfur (including oxides thereof such as sulfite and sulfate), nitrogen (e.g., $NR^1$ where $R^1$ is as defined above, and the like.) One preferred linker is a polyoxyalkylene of from 1 to 10 oxyalkylene units.

The term "detectible signal" refers to a signal that can be generated by the fluorescent imaging agent that is detectible either visually, spectroscopically, or by suitable software. It is understood that fluorescent signaling requires exposure of cells to an ultraviolet light source comprising the excitation wavelength of the fluorescent imaging agent so as to produce fluorescence.

The compounds of this invention may exist as solvates, especially hydrates. Hydrates may form during manufacture of the compounds or compositions comprising the compounds, or hydrates may form over time due to the hygroscopic nature of the compounds. Compounds of this invention may exist as organic solvates as well, including dimethylformamide, ether, and alcohol solvates among others. The identification and preparation of any particular solvate is within the skill of the ordinary artisan of synthetic organic or medicinal chemistry.

"Subject" refers to a mammal. The mammal can be a human or non-human animal mammalian organism.

"Treating" or "treatment" refers to the application of one or more pro-fluorescent compounds of this invention onto mammalian cells that are suspected of containing cancerous cells that express or overexpress the folic acid receptor so as to allow the clinician to confirm as to whether such cells are, in fact, such cancerous cells.

"Effective amount" refers to the amount of a compound of this invention that is sufficient to diagnostically evaluate whether a cell is cancerous or not.

The term "specificity" refers to the specific accumulation of a conjugate of this invention onto/into cancer cells at a rate sufficiently higher than non-cancerous or normal cells such that the fluorescence generated by such cancer cells is distinguishable from normal cells. In one embodiment, the conjugates of this invention accumulate at a rate of greater than 2:1 in cancer cells compared to non-cancerous cells. Preferably, the rate of accumulation is greater than 3:1, more preferably greater than 5:1, and even more preferably greater than 6:1 and most preferably at least 8:1. Accordingly the term "absorbed" refers to the surface accumulation or intracellular accumulation of the conjugates of this invention, although intracellular accumulation is desirable.

As used herein, the term "salts" of compounds disclosed herein include acid or base addition salts which retain the desired activity. When the compound of the present invention has a basic group, such as, for example, an amino group, salts can be formed with inorganic acids (such as hydrochloric acid, hydroboric acid, nitric acid, sulfuric acid, and phosphoric acid), organic acids (e.g., alginate, formic acid, acetic acid, benzoic acid, gluconic acid, glucuronic acid, fumaric acid, oxalic acid, tartaric acid, lactic acid, maleic acid, citric acid, succinic acid, malic acid, methanesulfonic acid, benzenesulfonic acid, naphthalene sulfonic acid, and p-toluenesolfonic acid) or acidic amino acids (such as aspartic acid and glutamic acid). When the compound of the present invention has an acidic group, such as for example, a carboxylic acid group, it can form salts with metals, such as alkali and earth alkali metals (*e.g.,* $Na^+$, $Li^+$, $K^+$, $Ca^{2+}$, $Mg^{2+}$, $Zn^{2+}$), ammonia or organic amines (*e.g.*, dicyclohexylamine, trimethylamine, trimethylamine, pyridine, picoline, ethanolamine, diethanolamine, triethanolamine) or basic amino acids (*e.g.,* arginine, lysine, and ornithine). Such salts can be prepared *in situ* during isolation and purification of the compounds or by separately reacting the purified compound in its free base or free acid form with a suitable acid or base, respectively, and isolating the salt thus formed.

Other fluorescent agents suitable for use in this disclosure are well known in the art some of which are depicted below. These agents can be modified into masked fluorescent agents that can be conjugated to folic or pteroic acid.

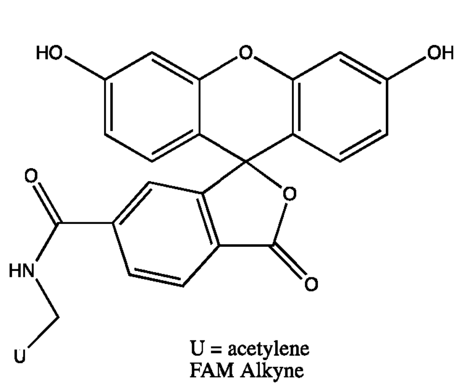

U = acetylene
FAM Alkyne

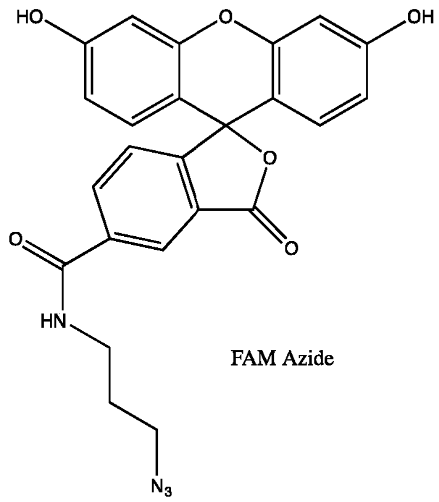

FAM Azide

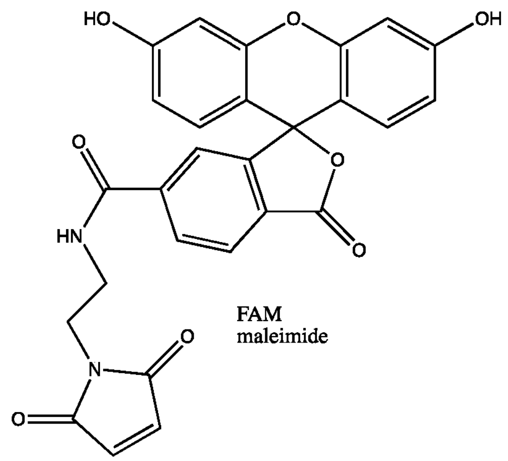

FAM
maleimide

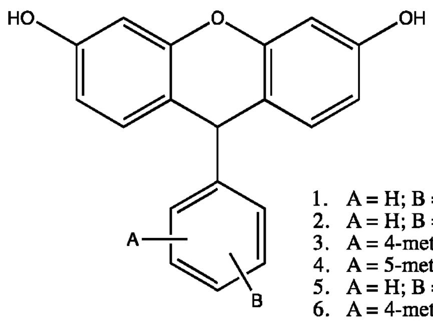

1. A = H; B = 2-COOH
2. A = H; B = 2-methyl
3. A = 4-methyl; B = 2-methyl
4. A = 5-methyl; B = 2-methyl
5. A = H; B = 2-methoxy
6. A = 4-methoxy; B= 2 methyl
7. A = 5-methyl; B = 2-methoxy
8. A = 4-methoxy; B = 2-methoxy
9. A = 5-methoxy; B = 2-methoxy

***6. Compounds***

This disclosure includes conjugates of the formula IA and IB: a conjugate of the formula IA and IB:

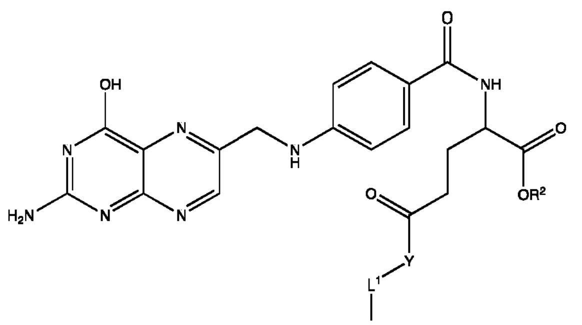

IA

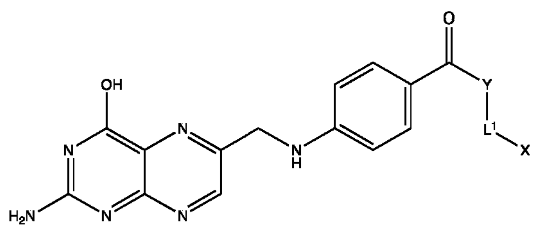

IB

where $L^1$ is a bond or a linker,
X is a masked imaging agent, and
Y is -O- or >$NR^1$ where $R^1$ is hydrogen, $C_1$-$C_4$ alkyl, substituted $C_1$-$C_4$ alkyl; $C_2$-$C_5$ alkenyl, substituted $C_2$-$C_5$ alkenyl, aryl, substituted aryl, cycloalkyl, substituted cycloalkyl, heteroaryl, substituted heteroaryl, heterocyclic, substituted heterocyclic;
$R^2$ is hydrogen or $C_1$-$C_4$ alkyl;
or salts, tautomers and/or solvates thereof,
provided that the conjugate is not

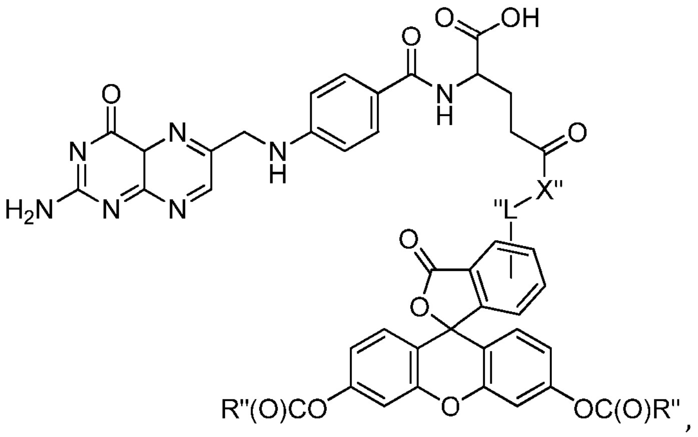

wherein X" is selected from the group consisting of -O- and >$NR^{20}$, L" is a bond or linking group having 1 to 30 atoms, R" is an alkyl group or a substituted alkyl group having from 4 to 30 carbon atoms and optionally 1 to 6 heteroatoms selected from oxygen, hydroxyl, sulfur, carbonyl, thiocarbonyl, chloro, bromo, iodo, and cyano; and $R^{20}$ is hydrogen or alkyl; or

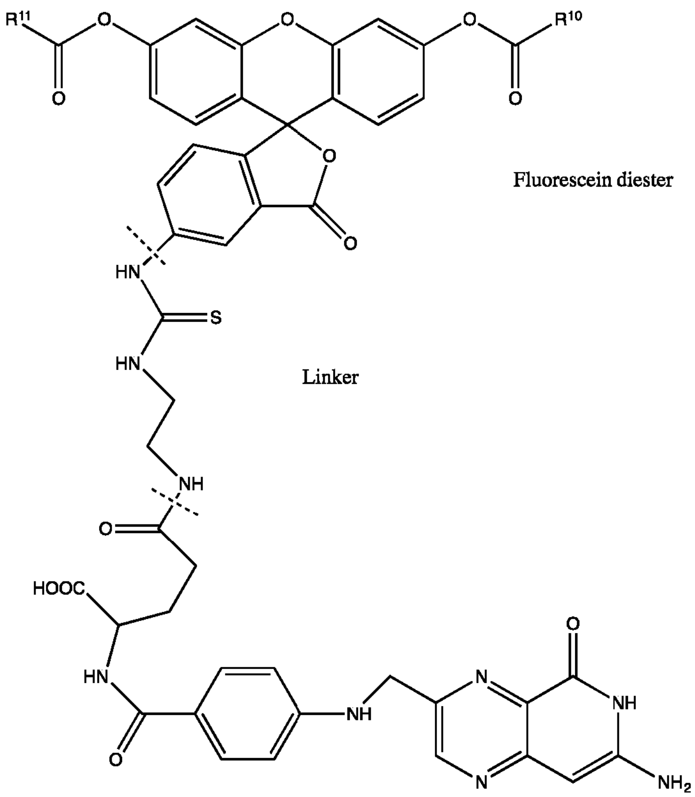

Fluorescein diester

Linker

Folic Acid

where $R^{11}$ and $R^{10}$ are $C_1$-$C_{30}$ ester groups; or

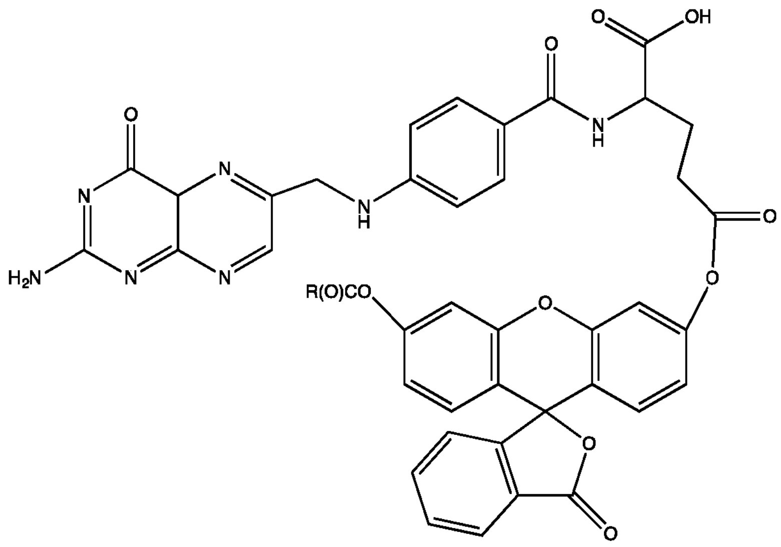

where R is an alkyl group or a substituted alkyl group having from 4 to 30 carbon atoms and optionally 1 to 6 heteroatoms selected from oxygen, hydroxyl, sulfur, thiol, carbonyl, thiocarbonyl, chloro, bromo, iodo, and cyano; or

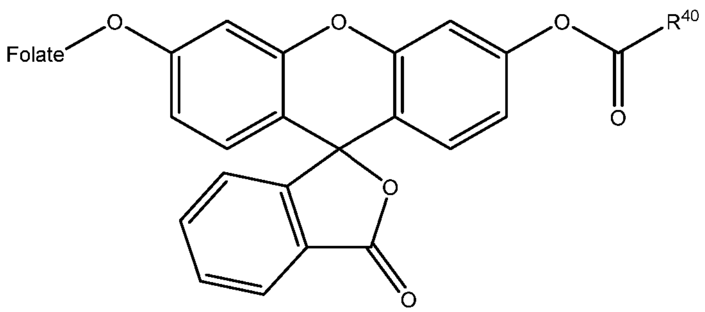

and

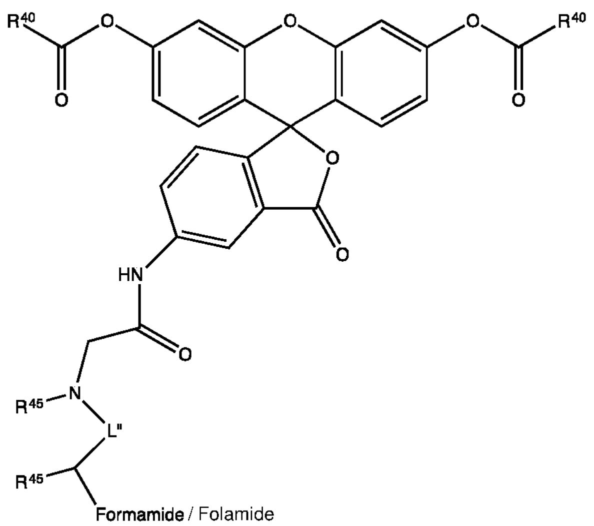

wherein $R^{40}$ is $C_1$-$C_6$ alkyl or heteroalkyl optionally substituted with a water solubility imparting functional group selected from the group consisting of hydroxy, methoxy, amino, carboxylic acid, and carboxylate;

$R^{45}$ is hydrogen or an optionally substituted alkyl group;

L" is a bond or a linking group having 1 to 30 atoms including -NHC(O)-alkylene-, -NHC(O)NH-alkylene-, -NHC(S)NH-alkylene-, and -OC(O)NH-;

folate is a folic acid moiety joined with the rest of the conjugate via an ester of the folic acid carboxyl group; and

folamide is a folic acid moiety joined with the rest of the conjugate via an amide of the folic acid carboxyl group.

## *7. Synthesis*

### General Synthetic Methods

The compounds of this invention can be prepared from readily available starting materials using the following general methods and procedures. It will be appreciated that where typical or preferred process conditions (i.e., reaction temperatures, times, mole ratios of reactants, solvents, pressures, etc.) are given, other process conditions can also be used unless otherwise stated. Optimum reaction conditions may vary with the particular reactants or solvent used, but such conditions can be determined by one skilled in the art by routine optimization procedures.

Additionally, as will be apparent to those skilled in the art, conventional protecting groups may be necessary to prevent certain functional groups from undergoing undesired reactions. Suitable protecting groups for various functional groups as well as suitable conditions for protecting and deprotecting particular functional groups are well known in the art. For example, numerous protecting groups are described in T. W. Greene and P. G. M. Wuts, Protecting Groups in Organic Synthesis, Third Edition, Wiley, New York, 1999, and references cited therein.

If the compounds of this invention contain one or more chiral centers, such compounds can be prepared or isolated as pure stereoisomers, i.e., as individual enantiomers or d(l) stereomers, or as stereoisomer-enriched mixtures. All such stereoisomers (and enriched mixtures) are included within the scope of this invention, unless otherwise indicated. Pure stereoisomers (or enriched mixtures) may be prepared using, for example, optically active starting materials or stereoselective reagents well-known in the art. Alternatively, racemic mixtures of such compounds can be separated using, for example, chiral column chromatography, chiral resolving agents and the like.

The starting materials for the following reactions are generally known compounds or can be prepared by known procedures or obvious modifications thereof. For example, many of the starting materials are available from commercial suppliers such as Aldrich Chemical Co. (Milwaukee, Wisconsin, USA), Bachem (Torrance, California, USA), Emka-Chemce or Sigma (St. Louis, Missouri, USA). Others may be prepared by procedures, or obvious modifications thereof, described in standard reference texts such as Fieser and Fieser's Reagents for Organic Synthesis, Volumes 1-15 (John Wiley, and Sons, 1991), Rodd's Chemistry of Carbon Compounds, Volumes 1-5, and Supplementals (Elsevier Science Publishers, 1989), Organic Reactions, Volumes 1-40 (John Wiley, and Sons, 1991), March's Advanced Organic Chemistry, (John Wiley, and Sons, 5th Edition, 2001), and Larock's Comprehensive Organic Transformations (VCH Publishers Inc., 1989).

## *8. Synthesis of Representative Compounds of the Invention*

In one embodiment, the method involves reacting a folic acid or pteroic acid with a fluorescent or pro-fluorescent (masked) compound. If a fluorescent compound is used, it must be post-treated after coupling to folic or pteroic acid to convert that compound to a pro-fluorescent or masked compound. Generally, however, it is preferred to combine a pro-fluorescent or masked compound having a reactive functionality that will form a covalent bond with folic or pteroic acid either directly or through a suitable linker.

In particular, the pro-fluorescent compounds used in the synthesis of conjugates of this invention are readily converted from fluorescent compounds using conventional methods. In one illustrative embodiment, fluorescein 5-isothiocyanate is converted to the corresponding diester via conventional esterification conditions as depicted below:

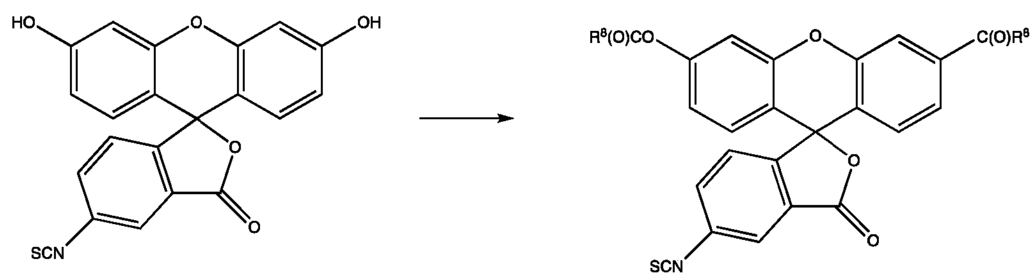

1 2

Scheme 1

where each $R^8$ is independently a $C_2$-$C_{20}$ group optionally containing 1 to 10 heteroatoms selected from -O-, -S-, carbonyl, >$NR^1$, -$NR^1R^1$, -OH, -SH, phosphate and sulfate. In particular, commercially available fluorescein 5-isothiocyanate,

compound 1, is combined with at least two equivalents and preferably an excess of a suitable carboxylic acid or acid anhydride in the presence of an excess of N,N-dicyclohexylcarbodiimide (DCC) and 4-dimethylaminopyridine (DMAP) in a suitable solvent or solvent mixture such as N,N-dimethylformamide/pyridine. The hydroxyl groups of fluorescein are readily esterified under such conditions. The reaction is typically conducted at a temperature of from about 0° to about 60°C for a period of time sufficient to allow for substantial completion of the reaction. Typically, such reactions are conducted for about 1 to 24 hours. Afterwards, the resulting product is recovered by conventional methods such as crystallization, column chromatography, HPLC, or precipitation. Reaction completion can be readily analyzed by loss of fluorescence in the reaction mixture.

It is understood that while compound 1 is illustrated as fluorescein 5-isothiocyanate, this compound is often a mixture with fluorescein 6-isothiocyanate and that the two compounds are deemed equivalents. As such, any reference to fluorescein 5-isothiocyanate is meant to include fluorescein 6-isothiocyanate. Likewise, the term "fluorescein" is intended to cover all known substitutions on the ring system for fluorescein such at dichloro substituted fluorescein compounds.

Separately, folic acid is converted to folic anhydride using methods well known in the art as described by Guaragna, et al., Bioconjugate Chemistry, 2012, 23:84-96 and especially at page 88 and as depicted below in Scheme 2. Specifically, folic acid, compound 3 is combined DCC in a solvent mixture of DMF (dimethylformamide) and pyridine (5:1). The reaction is conducted at a slightly elevated temperature of about 30°C although the reaction can be run at from about 0° to about 60°C. The reaction is continued until substantially complete and the product, folic anhydride - compound 4, can be recovered by conventional means such as chromatography, distillation, precipitation, high performance liquid chromatography (HPLC) and the like. Alternatively, the product can be used in the next step without purification and/or isolation.

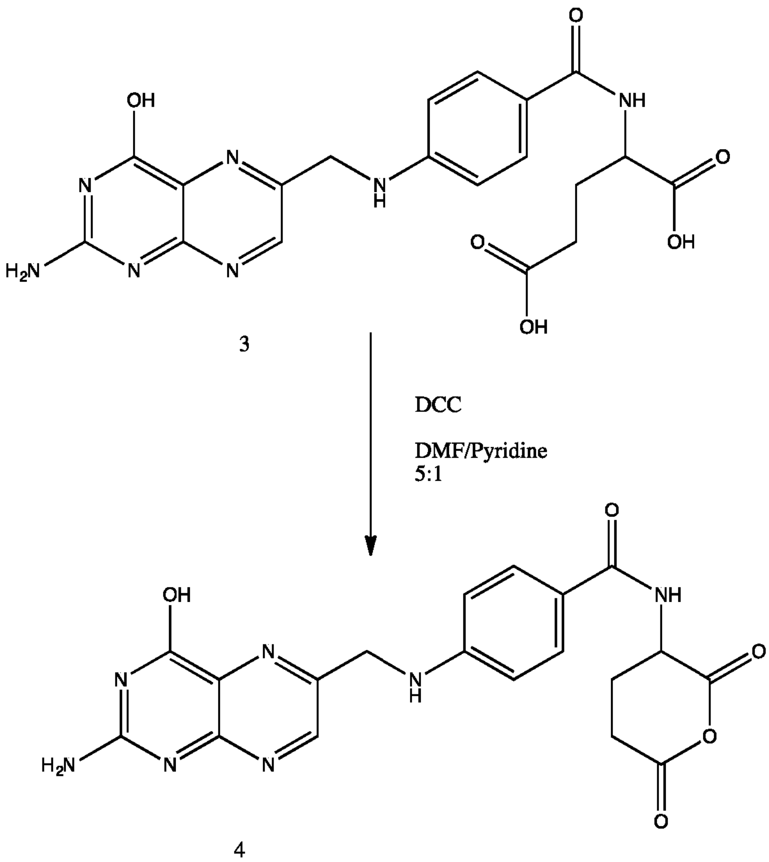


Scheme 2

Folic anhydride, compound 4, is next ring opened to form an amide linker moiety, compound 5, as shown in Scheme 3 below:

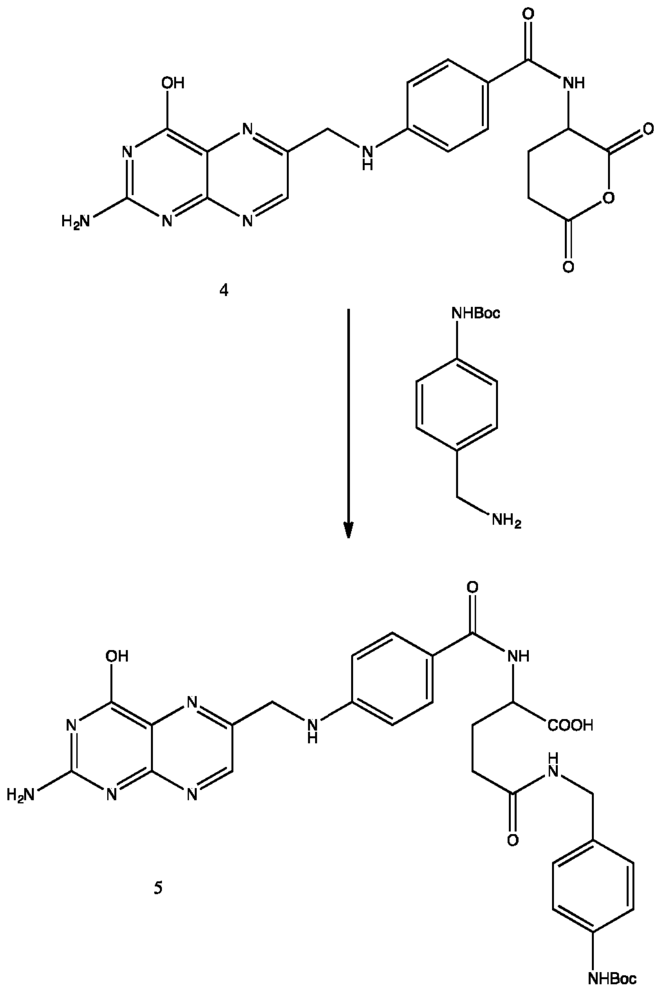

Scheme 3

Specifically, commercially available 4-aminomethyl-N-Boc-aniline is combined with folic anhydride, compound 4, together with dicyclohexylcarbodiimide (DCC) under conditions also set forth by by Guaragna, et al., Bioconjugate Chemistry, 2012, 23:84-96 to provide for compound 5. Removal of the Boc (t-butoxycarbonyl) protecting group proceeds via conventional methods using trifluoroacetic acid to provide for the free amino group (compound 6 - Scheme 4). The reaction is continued until substantially complete and the product, compound 4, can be recovered by conventional means such as chromatography, distillation, precipitation, HPLC, and the like. Alternatively, the product is used in the next step without purification and/or isolation.

In Scheme 3, the optional inclusion of a C1-C4 alcohol such as methanol leads to the corresponding ester.

Compound 6 is next linked to compound 2 to form compound (conjugate) 7 as shown in Scheme 4 below:

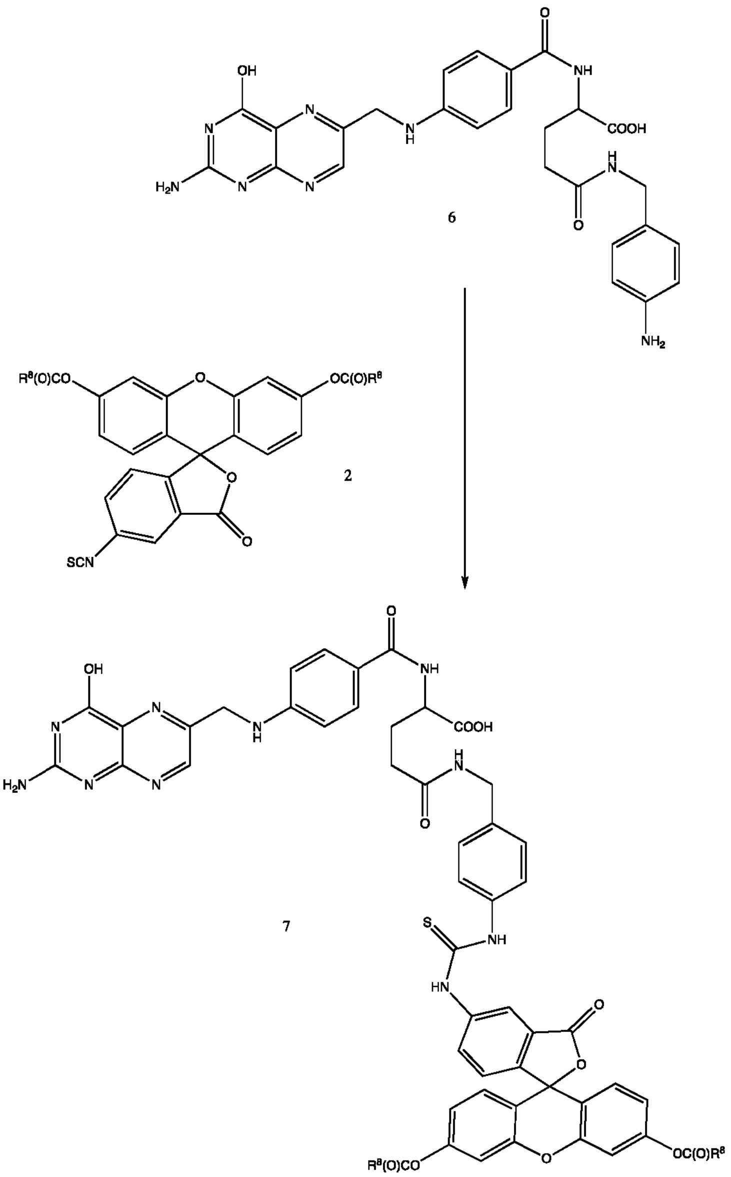

6

2

7

Scheme 4

Specifically, compound 2 and compound 6 are combined in a suitable inert aprotic solvent such as dimethylformamide (DMF), acetonitrile, methylene chloride, chloroform, ethyl acetate, tetrahydrofuran and the like. The reaction is typically conducted at from about 0° to about 50°C for period of time sufficient to substantially complete the reaction and preferably 1 to 24 hours. The reaction completion can be monitored by thin layer chromatography (TLC), high performance liquid chromatography (HPLC), and the product can be recovered by conventional methods such as chromatography, precipitation, crystallization, HPLC, and the like.

Alternatively, pteroic acid can be used in place of folic acid in the above reaction. The reaction conditions are substantially the same and the product is recovered in substantially the same manner. Note that the carboxyl group of pteroic acid will react similarly to the gamma carboxyl group of folic acid.

In the reaction schemes above, fluorescein based compounds can be replaced by non-fluorescein based compounds provided that such compounds are capable of having their fluorescence significantly reduced or eliminated by masking groups.

Still further, linkage of the folic acid to fluorescein can be accomplished through ester bond formation off of one of the phenolic alcohols as shown below:

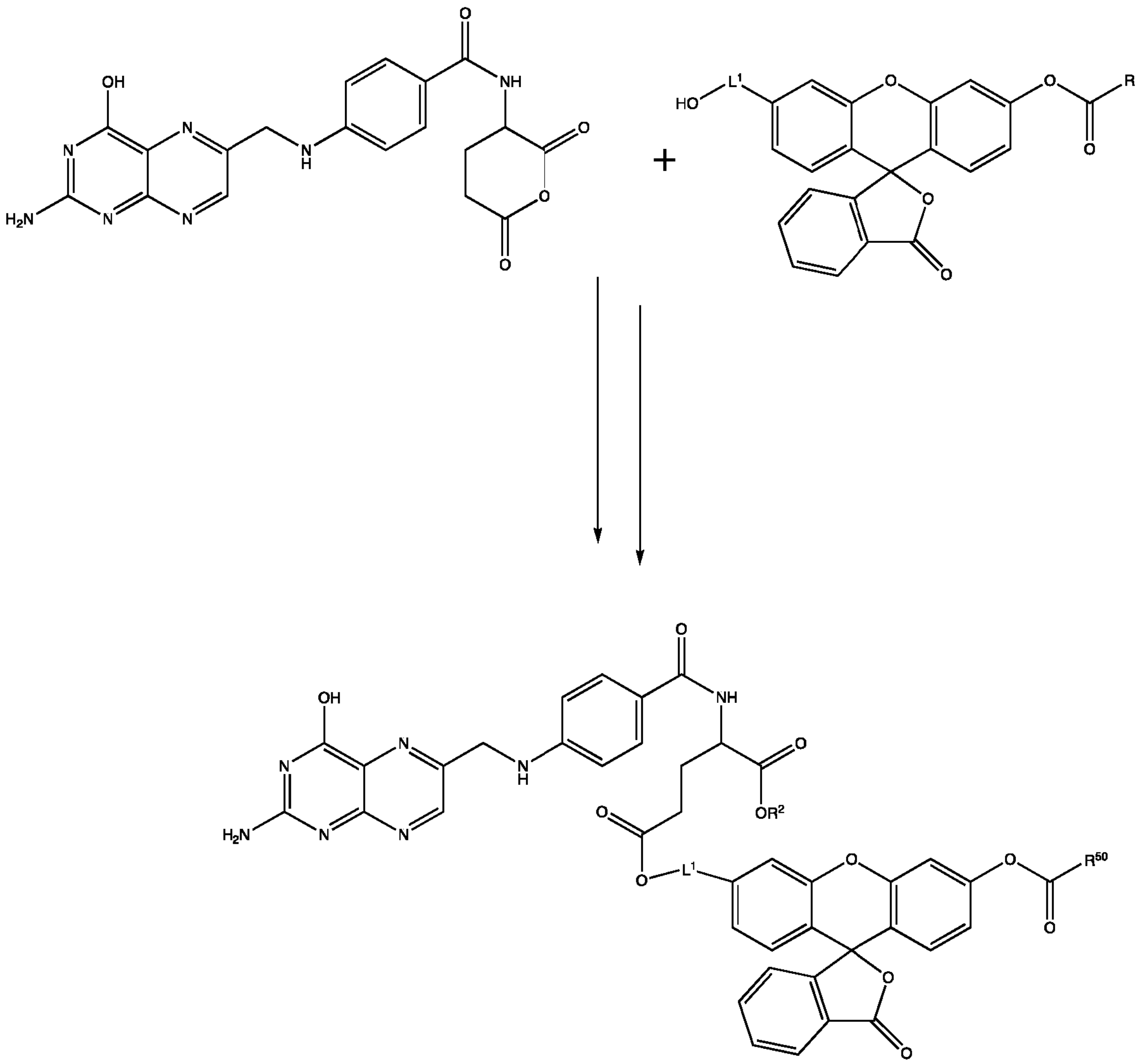

where $R^2$, $R^{50}$, and $L^1$ are as defined above.

Specifically, in the reaction above, folic anhydride (described above) is combined with fluorescein monoester wherein the remaining hydroxyl group is retained or extended via a linker using conventional techniques. In one embodiment, the linker is a polyoxyalkylene chain of from 1 to 20 units and, in one embodiment, that chain can be represented from left to right as $(CH_2CH_2O)_l$ where *l* is an integer of from 1 to 20. Introduction of such polyoxyalkylene chains or other linkers is well known in the art. Formation of the ester is also well known in the art and is described above albeit the formation of a sodium phenoxide derivative prior to ester formation will facilitate the reaction. Conversion of the alpha carboxyl group to an ester again proceeds via well known chemistry. In addition, the use of pteroic acid in this reaction in place of folic acid proceeds as above.

***9. Methods and Compositions***

The compounds of this invention are useful for detecting cancer cells in a cellular sample suspected of containing cancer cells and, preferably, cancer cells that overexpress folic acid receptors. Many cancers overexpress these receptors including but not limited to ovarian, cervical, epithelial, breast, colorectal, brain, lung, and kidney (renal) cancers. These compounds are applied to a cellular sample suspected of containing cancer cells such as those recited above. Application can be made via any conventional route such as intermixing the cellular sample with a liquid formulation of one or more compounds of this invention. For example, a sterile aqueous solution of a conjugate of this invention can be sprayed onto a cellular mass such as a surgical site or "painted" onto such a site. Alternatively, the compositions can be targeted to specific sites of the surgical field that the clinician wishes to evaluate for cancer cells.

In particular, the compounds/conjugates of this invention uniquely involve a masking group that blocks the label portion of the conjugate from signaling. The masking group is rapidly removed when introduced into cancer cells but remains intact in the extracellular space. In one embodiment, the label portion is a fluorescein compound or derivative

thereof and the masking groups are esters on each of the alcohol groups on the fluorescein. Without being limited to any theory, blocking of the signalizing of these fluorescein compounds arises because esterification locks these compounds into a structure that is incapable of generating a fluorescent signal. Still further, once absorbed, intracellular esterases and other enzymes remove these ester functionalities on the label thereby restoring its labeling properties. Moreover, these conjugates exhibit directional capacity to selectively bind to and be absorbed by folate dependent cancers. That is to say that the folic acid or pteroic acid moiety on the conjugates allow for selective binding to cancer cells that have folic acid receptors and in particular excess folic acid receptors as compared to normal cells.

When so used, the compounds of this invention will be administered in a diagnostically effective amount by any of the accepted modes of administration for agents that serve similar utilities. The actual amount of the compound of this invention, i.e., the active ingredient, will depend upon numerous factors such as the size of the surgical site of the subject, the signal strength of the unmasked compound used, the route and form of application, and other factors well-known to the skilled artisan. In one particular embodiment, the surgical oncologist can reapply the compound/compositions described herein multiple times in order to identify remnant cancer cells.

This disclosure is not limited to any particular composition or carrier, as such may vary. In general, compounds of this invention will be administered as aqueous compositions. Such compositions comprise water and optionally a biologically compatible amount of a cosolvent such as ethanol, DMSO, ethyl lactate, and the like. For example, a solvent mixture of 9:1 water : DMSO provides a suitable solution of a compound of this invention to be applied to cells or tissues. In one embodiment, such compositions comprise from about 0.00001 to about 90 weight percent of a compound of this invention and preferably about 0.001 to about 10 weight percent and even more preferably from about 0.001 to 1 weight percent.

Suitable compositions comprising compounds of this invention may be manufactured by any of the methods well-known in the art, such as, for example, by conventional mixing, dissolving, melting, emulsifying, and the like.

## *10. Formulation Examples*

The following are representative pharmaceutical formulations containing a compound of this invention.

Formulation Example 1 -- Suspension formulation

The following ingredients are mixed to form a suspension for oral administration.

| Ingredient | Amount |
|---|---|
| compound of this invention | 10 mg |
| colorings | 0.5 mg |
| distilled water | q.s. to 100 mL |

Formulation Example 2 -- Injectable formulation

The following ingredients are mixed to form an injectable formulation.

| Ingredient | Amount |
|---|---|
| compound of this invention | 0.2 mg-20 mg |
| sodium acetate buffer solution, 0.4 M | 2.0 mL |
| HCl (1N) or NaOH (1N) | q.s. to suitable pH |
| water (distilled, sterile) | q.s. to 20 mL |

## *11. EXAMPLES*

This invention is further understood by reference to the following examples, which are intended to be purely exemplary of this invention.

The following abbreviations are used in the examples below and have the following meanings. If an abbreviation is not defined, it has its art recognized meaning. In addition, all temperatures are in degrees Celcius unless otherwise noted.

DCC = dicyclohexycarbodiimide

DMF = N,N-dimethylformamide

DMAP = N,N-dimethylaminopyridine

DMSO = dimethylsulfoxide

eq. = equivalents

ether = diethyl ether

FBS = fetal bovine serum

mg = milligram

mL = milliliter

mm = millimeter

mM = millimolar

mmoles = millimoles

RPMI = Roswell Park Memorial Institute medium

MP = melting point

RT = room temperature

TFA = trifluoroacetic acid

TLC = thin layer chromatography

μM = micromolar

μm = micromoles

V/V = volume / volume

***Example 1*** - Synthesis of 2-(4-(((2-amino-4-oxo-3,4dihydropteridin-6-yl)methyl)amino)benzamido)-5-((3'6'-dihydroxy-3-oxo-3H-spiro[isobenzofuran-1,9'-xanthen]-5-yl)amino-5-oxopentanoic acid (compound 13)

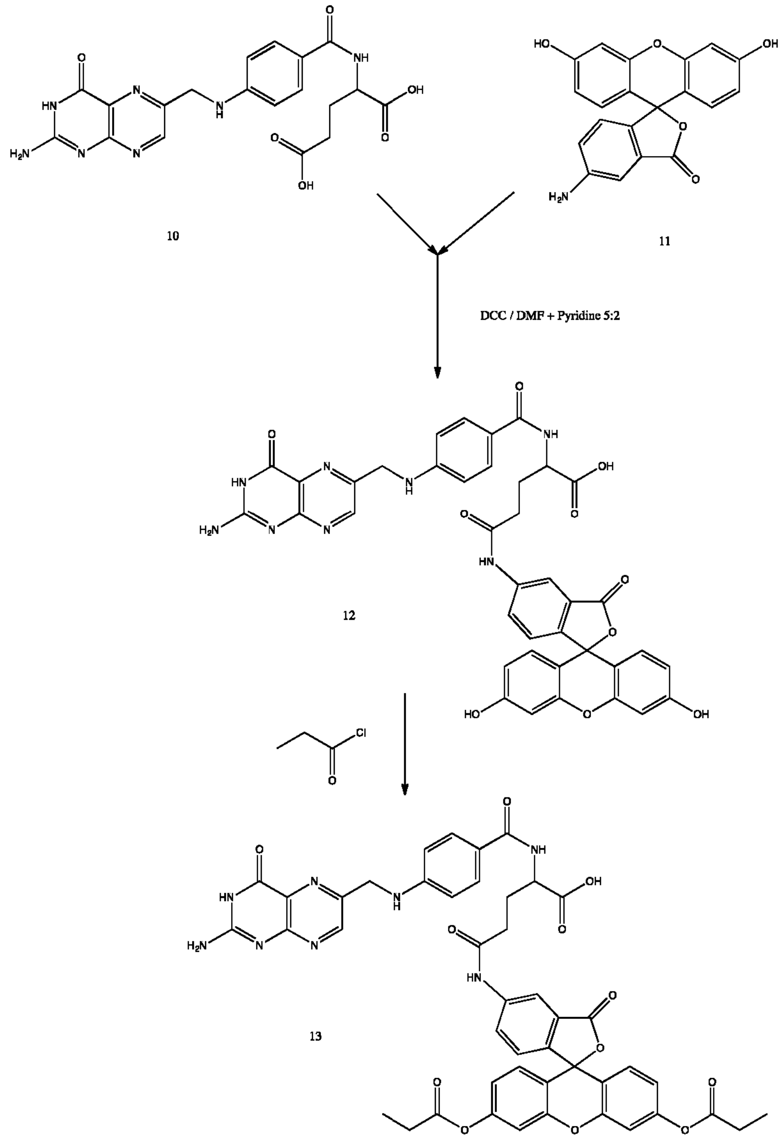

10
11
12
13

A mixture of 100 mg (0.22 mmoles) folic acid (compound 10) in an anhydrous 20 mL DMF solution plus 4 mL pyridine was heated and vigorously shaken to get a clear golden solution. To this solution was added 6 equivalents of DCC (280 mg, 1.36 mmoles). The reaction mixture was mixed in an ultrasound bath in the dark for 15 minutes twice, while the bath warmed to about 29°C. The resulting cloudy solution was added to a flask containing 1.1 equivalents 5-aminofluorescein (86.5mg, 0.249 moles) (compound 11). The resulting reaction mixture was wrapped with aluminum foil and stirred at room temperature overnight. After about 18 hours the mixture was filtered through celite and added drop-wise to a mixture of 70 mL ether and 30 mL acetone. The cloudy mixture was stored in the dark in a freezer for several days. The solid (compound 12) was filtered off, washed with ether and air-dried to a constant mass of 115.7 mg (66.3%).

29.4 mg of the folic acid-fluorescein conjugate (compound 12) was dissolved in 2 mL dry DMF, and 6 equivalents (23.6 mg) triethylamine was added, followed by 5 equivalents of propionyl chloride (17.6 mg). The reaction was stirred at room temperature for several days, and poured into a mixture of water and ethyl acetate. The organic layer was washed with water and brine and dried over sodium sulfate. After evaporation of the solvent the solid was further dried to yield 20.6 mg (54%). MP: >290°. Confirmation of the product was further provided by loss of fluorescence due to the diesterification of the fluorescein phenolic hydroxyl groups of compound 13. Specifically, when a sample of compound 13 was dissolved in methanol a non-fluoresecent solution resulted. On treatment with a few drops of ammonia water intense fluorescence was noted. The ammonia is a strong deacylating agent that unmasks the masked fluorescent fluorescein diester.

***Example 2*** - Synthesis of *O*,*O*'-(5-(3-(4-((4-(4-(((2-amino-4-oxo-3,4-dihydropteridin-6-yl)methyl)amino)benzamido)-5-methoxy-5-oxopentanamido)methyl)phenyl)thioureido)-3-oxo-3*H*-spiro[isobenzofuran-1,9'-xanthene]-3',6'-diyl) bis)(2-methoxyethyl) disuccinate (compound 18)

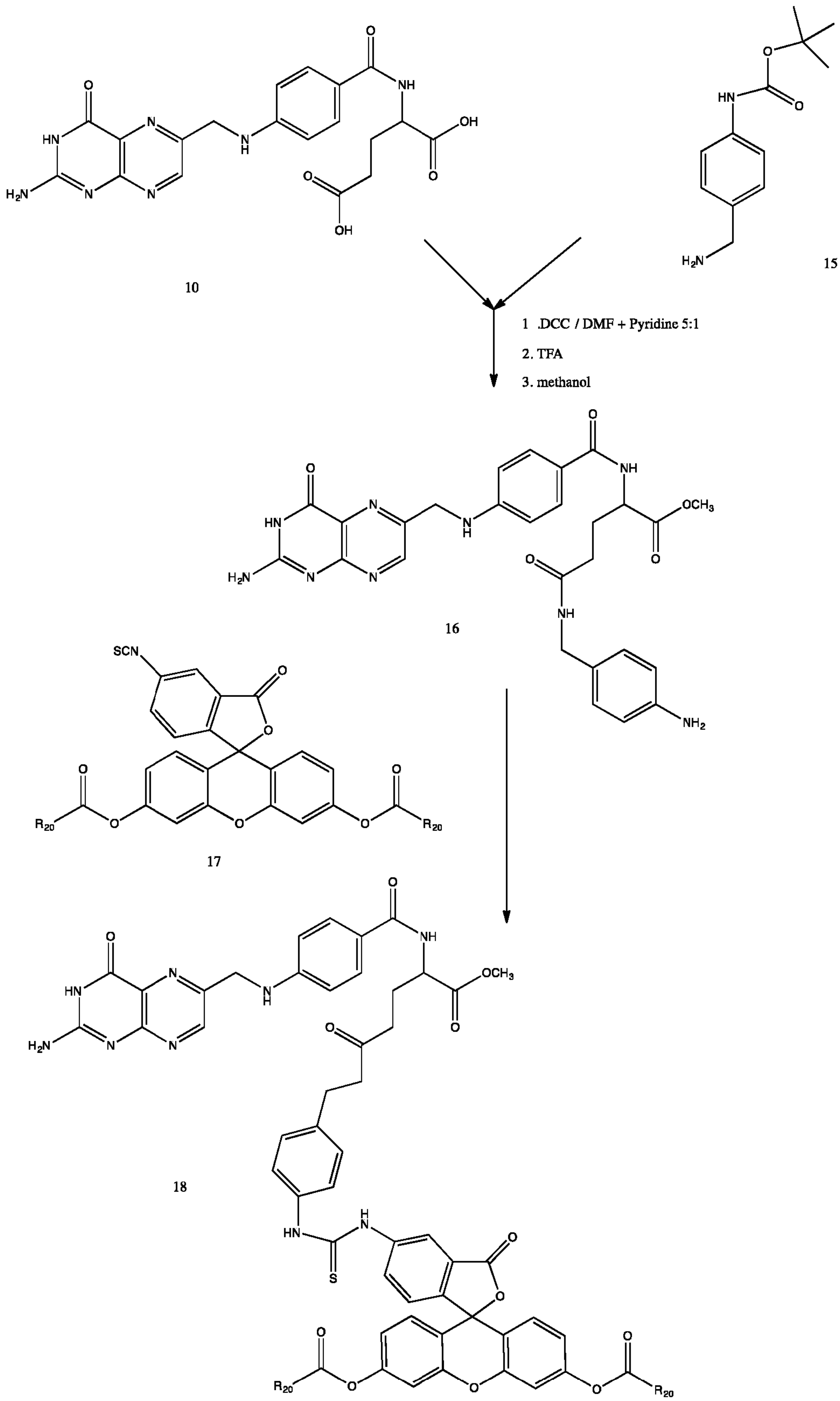


10

15

16

17

18

A. Synthesis of O,O'-5-isothiocyanato-3-oxo-3H-spiro[isobenzofuran-1,9'-xanthene]-3',6'-diyl) bis(2-methoxyethyl)disuccinate (compound 17) A1.

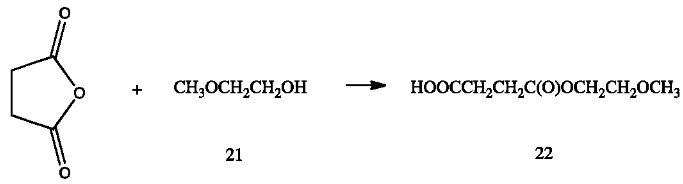


20 21 22

The above reaction follows the literature preparation described by J. Materials Chemistry, 2014, 2(26):4142-4145. Specifically, a slight excess of succinic anhydride was combined with 2-methoxyethanol in methylene chloride in a flask at about 20° C. A solution of triethylamine in methylene chloride was added dropwise over about a 15 minute period during which the reaction produced sufficient heat so that the solvent began to boil. Afterwards, the addition of triethylamine was stopped and the reaction stirred overnight after returning to room temperature.

The reaction was stopped and the reaction solution washed with brine and the organic layer was recovered. The solvent was stripped and the resulting product was purified by column chromatography (silica gel using a gradient of from 0 to 10% methanol in methylene chloride v/v). The resulting product (compound 12) was used as is without further purification or isolation. A2

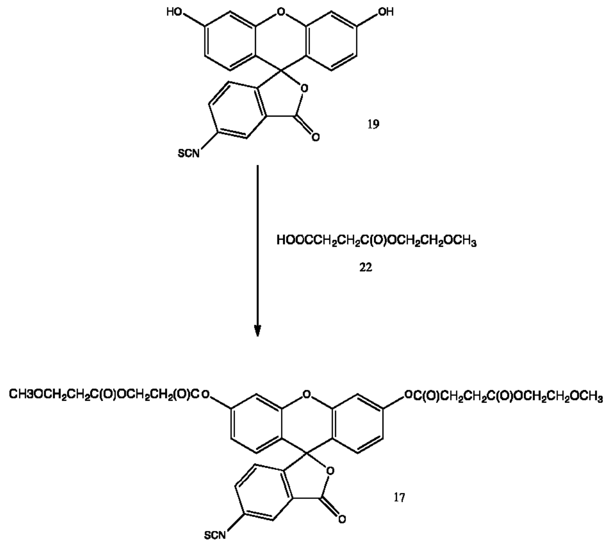


19 22 17

Approximately 1 eq. of compound 22 was dissolved in methylene chloride and then combined with approximately 1 eq. of DCC at room temperature. The mixture was stirred for approximately 5 minutes and then 0.25 equivalents of DMAP and approximately 0.25 eq. of fluorescein were added thereto. The reaction mixture was then sonicated at 26°C until the suspension was substantially dissipated which occurred over approximately 15 minutes. The resulting reaction mixture was stirred overnight at room temperature and monitored for reaction completion by TLC. Upon substantial reaction completion, the non-soluble components were filtered and the resulting solution was placed on a silica column for purification purposes. The column was eluted with a solvent gradient starting at 0% methanol and 100% methylene chloride and finishing with 10% methanol and 90% methylene chloride (v/v). The elutant containing the desired compound was stripped of solvent and the resulting compound 14 was substantially free of fluorescence indicative of formation of diester. A small aliquot of the compound was contacted with a sodium hydroxide solution that immediately provided for fluorescence indicative of deacylation. The sodium hydroxide is a strong deacylating agent that unmasks the masked fluorescent fluorescein diester.

A mixture of 100 mg (0.22 mmoles) folic acid (compound 10) in an anhydrous 20 mL DMF solution plus 4 mL pyridine was heated and vigorously shaken to get a clear golden solution. To this solution was added 6 equivalents of DCC (280 mg, 1.36 mmoles). The reaction mixture was mixed in an ultrasound bath in the dark for 15 minutes twice, while the bath warmed to 29°C. The resulting cloudy solution was added to a flask containing 1.1 eq. *tert-butyl* (4-aminomethyl) phenyl)-carbamate (58.3 mg) (compound 15). The resulting reaction mixture was wrapped with aluminum foil and stirred at room temperature overnight. After about 18 hours 1mL of methanol was added to esterify the alpha-carboxylic acid (this is an optional step). After stirring for an additional 24 hours at RT, the mixture was filtered through celite and added drop-wise to a mixture of 70 mL ether and 30 mL acetone. The cloudy mixture was stored in the dark in a freezer for several days. The solid was filtered off, washed with ether and air-dried to a constant mass of 72 mg (48.2%). Without further purification, 24 mg of this compound was added to 0.5 mL TFA (large excess) stirred at room temperature and then stored in the

refrigerator. The TFA was evaporated to yield 20 mg (35.7 μm) of compound 16. This material was then dissolved in a small amount of DMF and treated with 1 equivalent (25 mg) of compound 17. The reaction was stirred at room temperature overnight. The mixture was added to a large amount of ether (75 mL) to yield a precipitate, which was filtered off, washed with ether and dried to 29 mg (64%) of a tan solid, MP: softens at 200°C and melts at 216-226°C (compound 18) A small sample of this material was dissolved in methanol yielding a clear colorless solution. When a few drops of ammonia water were added, the solution became intensely fluorescent. The ammonia is a strong deacylating agent that unmasks the masked fluorescent fluorescein diester.

***Comparative Example A***

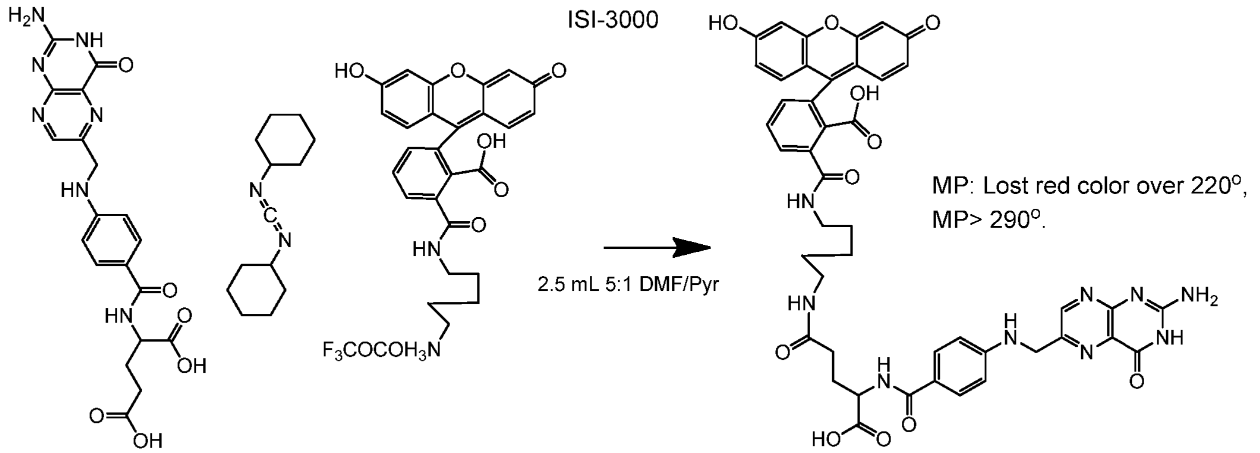


25

Compound 25 was prepared following the procedures set forth above ad provided for the title compound as a comparative example (no ester groups on the fluorescein moiety).

***Example 3*** - Synthesis of the fluorescein diester, compound 26

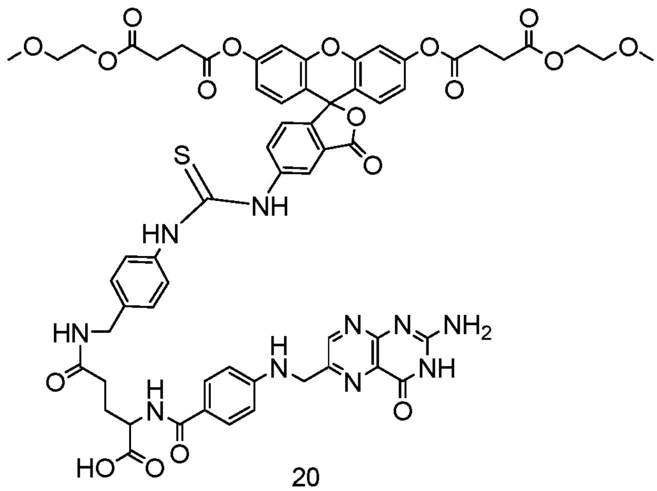

20

Following the procedure of Example 2 and omitting the addition of methanol, compound 20 is prepared. When a sample of compound 13 was dissolved in methanol, a non-fluoresecent solution resulted. On treatment with a few drops of ammonia water intense fluorescence was noted. The ammonia is a strong deacylating agent that unmasks the masked fluorescent fluorescein diester.

***Example 4*** - Synthesis of the fluorescein diester, compound 27

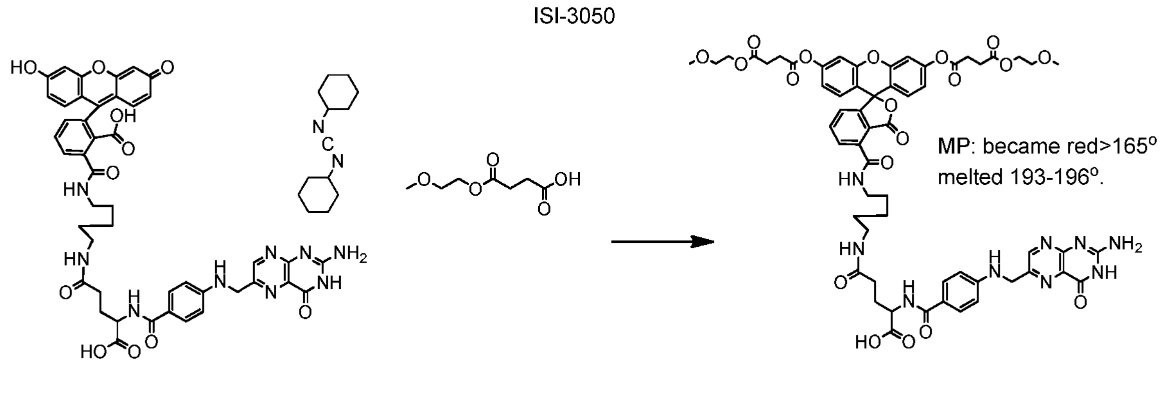


27

Compound 27 was prepared following the procedures set forth above. Specifically, when a sample of compound 13 was dissolved in methanol a non-fluoresecent solution resulted. On treatment with a few drops of ammonia water intense fluorescence was noted. The ammonia is a strong deacylating agent that unmasks the masked fluorescent fluorescein diester.

***Example*** *5* - Synthesis of Pteroic Acid Derivative

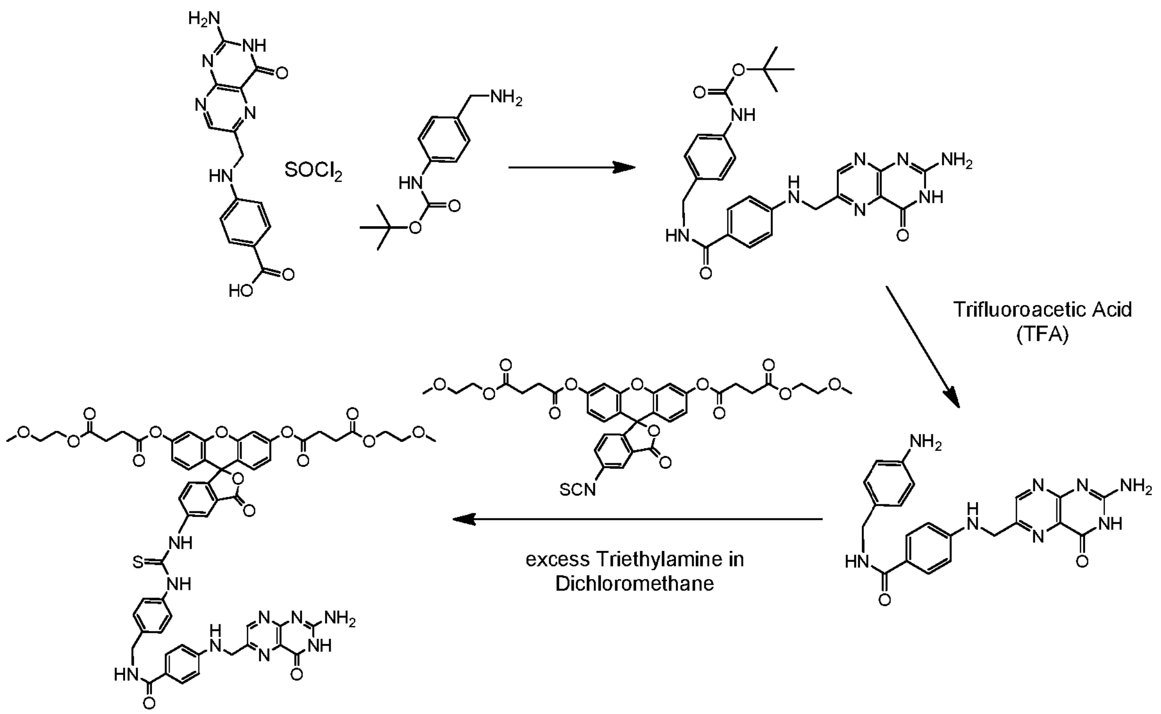


12 mg of pteroic acid (Sigma Aldrich, St. Louis, Missouri, USA) was mixed with about 1.5 mL of DMF, and heated to about 80°C with stirring for a few minutes. The pteroic acid did not go into solution. The mixture was then cooled to RTand excess thionyl chloride was added and a clear solution was generated almost immediately indicating the formation of the acid chloride. 8.2 mg 4-(aminomethyl) t-Boc-aniline was added and the clear solution stirred overnight. The clear golden-colored solution was treated with a few drops of triethylamine and the solution became very dark and viscous. After stirring

for 3 hours, the solution was added to 50 mL of ether using several milliliters of acetone in the transfer. The precipitate was filtered and was dark and wet. It was treated with charcoal and washed through the funnel with DMF and then acetone. The filtrate was again added to 50 mL of ether and a lighter colored precipitate formed. The mixture was stored in a refrigerator overnight. The solid was filtered off and treated with excess trifluoroacetic acid (TFA) to remove the Boc group. Dichloromethane was used as the solvent. The excess TFA was removed under vacuum and the residue was dissolved in dichloromethane and excess triethylamine added until the solution was basic. To this solution was added excess fluorescein 5-isothiocyanate diester (as depicted in the scheme above) in dichloromethane. The reaction was then stirred at room temperature. The solution was then added to 10 mL of ether that was then cooled in ice. The solid was collected after centrifuging and transferring to a glass fritted filter funnel, washed with excess ether, and air-dried. About 8mg of the product was obtained as a dark solid.

$$MP = 155\text{-}158°C$$

To confirm that the product contained the fluorescein diester moiety, a sample of the product was dissolved in methanol to provide a clear amber solution with no evidence of fluorescein fluorescence. Upon addition of aqueous ammonia, an intense characteristic yellow-green fluorescence was obtained.

## ***Biological Examples***

### ***A. Detection of Ovarian Cancer Cells***

Compound 24 and comparative compound A were evaluated for their ability to be absorbed by cancer cells and then, in the case of compound 24, deacylated by intracellular esterases so as to regenerate a fluorescent structure. Specifically, approximately 500,000 SDOV3 cells (an ovarian cancer cell line) were seeded into separate 35 mm culture dishes containing a folate-free growth medium (RPMI + 10% FBS). The next day, the medium was replaced with a folate-free medium (no FBS). In one culture dish, the medium was supplemented with 25 micromolar of compound 24; and, in another culture dish, the medium was supplemented with 50 micromolar of comparative compound A. After incubation, the cells were washed with HBSS (Hank's balanced salt solution) to remove unbound compound. The cells were then imaged with a 20x immersion objective on a standard upright fluorescent microscope. In the case of compound 24, the fluorescent signal was clear, consistent and unambiguous evidencing that cancer cells were fluorescent and that the fluorescent signal was not evident in the solution. Fig. 1 illustrates a picture showing the fluorescence generated. Note that only the cancer cells evidenced fluorescence and that the solution remained non-fluorescent. As to comparative compound A, that solution showed a burst of fluorescence but immediately the fluorescence was bleached and the composition no longer was capable of fluorescing. This evidences that that composition was not suitable for use in the methods described herein.

These results establish that compound 24 targeted cancer cells, were absorbed by cancer cells, and were deacylated by intracellular enzymes. The persistent signaling solely in the cancer cells evidenced that deacylated compound 24 did not efflux from the cancer cells. On the other hand, comparative compound A also was absorbed by the cancer cells and immediately fluoresced but that was followed by loss of fluorescence likely due to bleaching under the intense light used.

Taken together, the compounds of this invention are suitable for use in detecting remnant cancer cells. Because certain cancer cells preferentially uptake the conjugates of this invention, after incubation for a period of time, removal of the applied solution from the surgical field will limit absorption into normal cells. Such can be accomplished under conventional lavage/washing conditions.

### ***B. Detection of Ovarian Cancer Cells is via the Folic Acic Receptor***

This example establishes that compound 24 is specific for the folate receptor. Specifically, compound 24 was used in a folate free medium and in a medium using excess folate that competed with compound 24. The rationale is that in the presence of excess folate, compound 24 would have to compete for binding to the folate binding protein and therefore there would be less signal than when compound 24 was the sole source of folate.

To test this hypothesis, SKOV3 cells were incubated with 10 μM of compound 24 or with 10 μM of compound 24 + 1 mM folate (100 xs). Cells were then washed and imaged as before.

All images were analyzed using exactly the same parameters. Mean intensity was measured from identical regions with in each image. Images, shown in Figure 2A and 2B, clearly indicate that folate competes with compound 24 for labeling ovarian cancer cells. These images establish that compound 24 binds to cells through the folate binding protein and not by some other route.

### *C. Dose Response*

This experiment establishes that compound 24 provides a dose response relative to the fluorescence generated. Specifically, SKOV3 cells were incubated with 10 μM, 25 μM, or 50 μM of compound 24 in RPMI supplemented with 0.25% BSA. Cells were incubated for 1 hour then washed with HBSS and imaged as before.

All images were analyzed using exactly the same parameters using the image J software suite. Mean intensity was measured from identical regions with in each image. Images, shown below, show a clear dose response for compound 124 in labeling cells. The bar graph shows the mean intensity of each image and supports the conclusions from visual inspection of the images. While BSA was used as a supplement, acylated BSA may be more practical.

The results of this experiment are provided below:

| | 10 μM | 25 μM | 50 μM |
|---|---|---|---|
| Mean | 312.8 | 360.4 | 545.5 |
| Standard error | 2.3 | 9.3 | 1.6 |

These results demonstrate a dose dependent response.

## Claims

1. The conjugate of the formula:

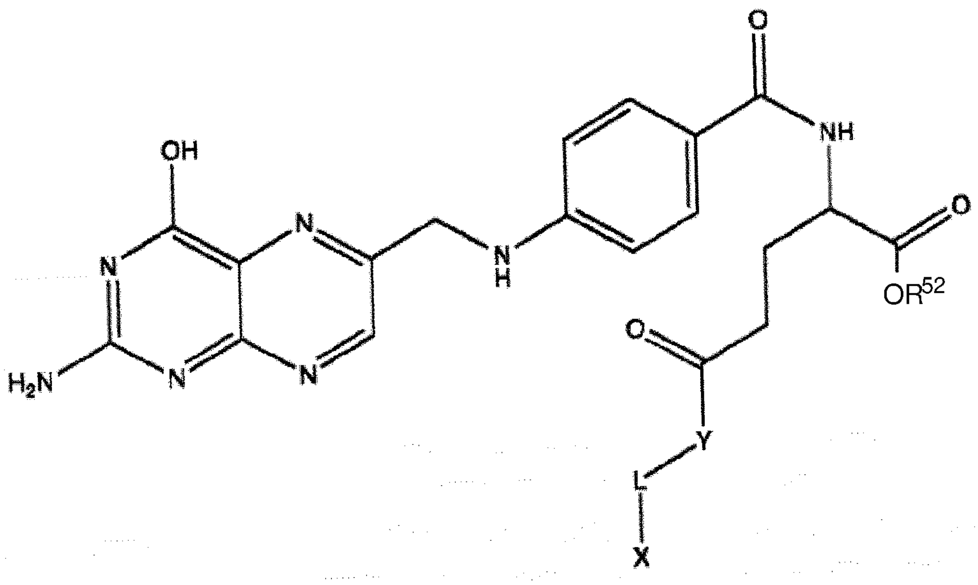

where $R^{52}$ is H or $CH_3$; and

| Y | L (from Y to X) | X | Imaging Agent |
|---|---|---|---|
| NH | $-(CH_2CH_2O)_3CH_2CH_2NH-CH_2C(O)NH-$ | 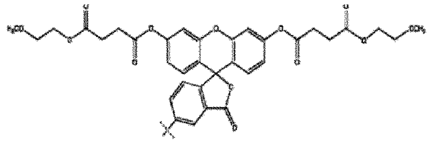 | di(methoxy-ethoxy) carbonylethyl carbonyloxy) 5-fluorescein |
| NH | $-(CH_2CH_20)_3CH_2CH_2NH-C(S)NH-$ | 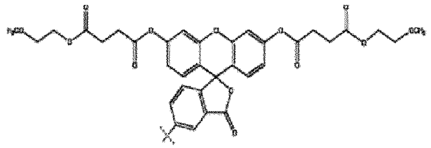 | di(methoxy-ethoxy) carbonylethyl carbonyloxy) fluorescein |
| NH | $-CH_2CH_2-$ | 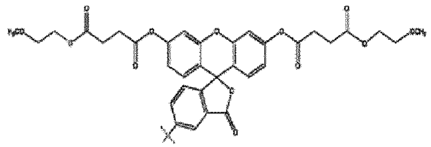 | di(methoxy-ethoxy) carbonylethyl carbonyloxy) 5-fluorescein |
| O | $-(CH_2CH_2O)_3CH_2CH_2O-C(S)NH-$ | 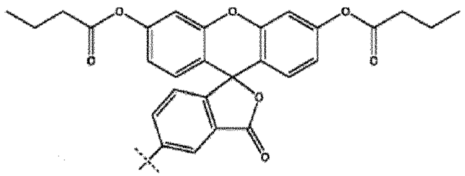 | di-(*n*-butyl-carbonyloxy) 5-fluorescein |
| NH | $-(CH_2CH_2O)_3CH_2CH_2O-C(S)NH-$ | 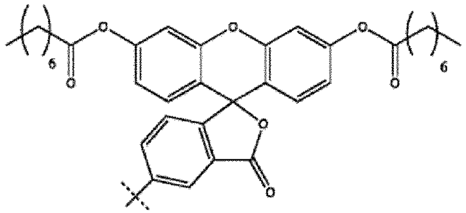 | di-(n-hexacarbonyloxy) 5-fluorescein |
| NH | $-CH_2CH_2-$ | 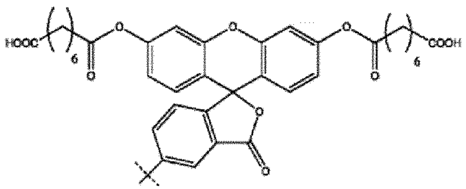 | di-(8-carboxy-hexacarbonyloxy) 5-fluorescein |

(continued)

| Y | L (from Y to X) | X | Imaging Agent |
|---|---|---|---|
| NH | $-(CH_2CH_2O)_3CH_2CH_2NH-CH_2C(O)NH-$ | 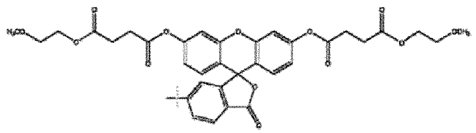 | di(methoxy-ethoxy) carbonylethyl carbonyloxy) 6-fluorescein |
| NH | $-(CH_2CH_2O)_3CH_2CH_2NH-C(S)NH-$ | 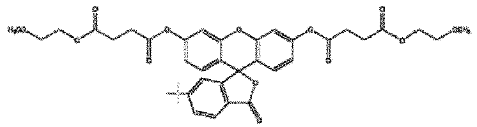 | di(methoxy-ethoxy) carbonylethyl carbonyloxy) 6-fluorescein |
| NH | $-CH_2CH_2-$ | 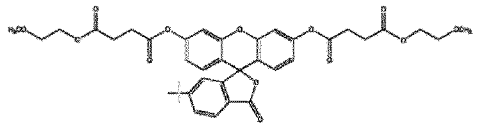 | di(methoxy-ethoxy) carbonylethyl carbonyloxy) 6-fluorescein |
| O | $-(CH_2CH_2O)_3CH_2CH_2O-C(S)NH-$ | 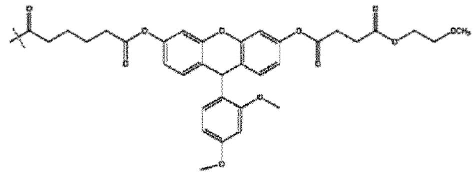 | |
| NH | $-(CH_2CH_2O)_3CH_2CH_2O-C(S)NH-$ | 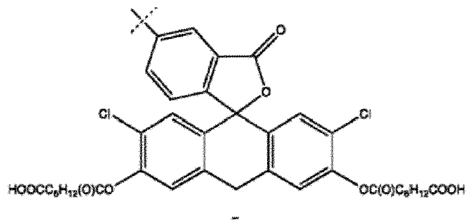 | 5',7'-dichloro-di-(8-carboxy-hexacarbonyloxy) 5-fluorescein |
| NH | $-(CH_2)_5-$ | 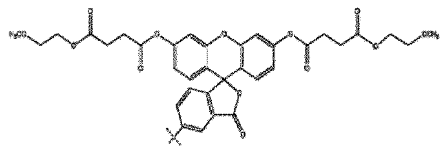 | di(methoxy-ethoxy) carbonylethyl carbonyloxy) 5-fluorescein |

or salts, tautomers and/or solvates thereof.

**2.** The conjugate of the formula:

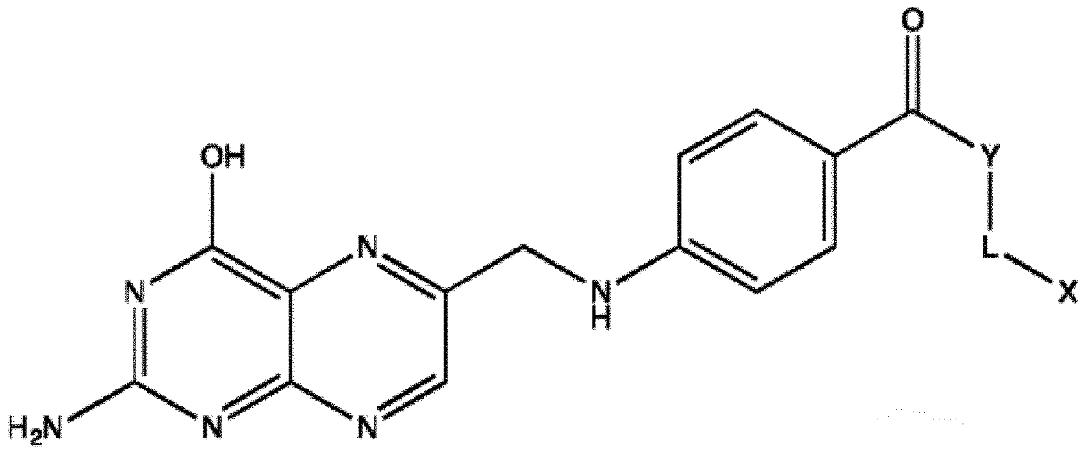

Where

| Y | L (from Y to X) | X | Imaging Agent |
|---|---|---|---|
| NH | $-(CH_2CH_2O)_3CH_2CH_2NH-CH_2C(O)NH-$ | 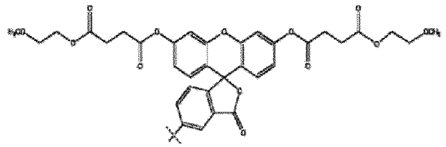 | di(methoxy-ethoxy) carbonylethyl carbonyloxy) 5-fluorescein |
| NH | $-(CH_2CH_2O)_3CH_2CH_2NH-C(S)NH-$ | 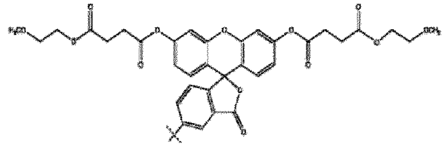 | di(methoxy-ethoxy) carbonylethyl carbonyloxy) fluorescein |
| NH | $-CH_2CH_2-$ | 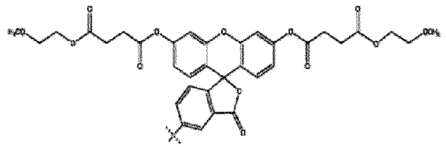 | di(methoxy-ethoxy) carbonylethyl carbonyloxy) 5-fluorescein |
| O | $-(CH_2CH_2O)_3CH_2CH_2O-C(S)NH-$ | 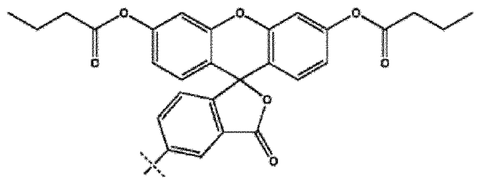 | di-(*n*-butyl-carbonyloxy) 5-fluorescein |
| NH | $-(CH_2CH_2O)_3CH_2CH_2O-C(S)NH-$ | 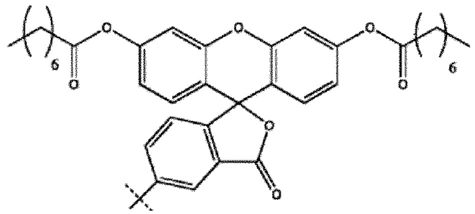 | di-(*n*-hexa-carbonyloxy) 5-fluorescein |

(continued)

| Y | L (from Y to X) | X | Imaging Agent |
|---|---|---|---|
| NH | $-CH_2CH_2-$ | 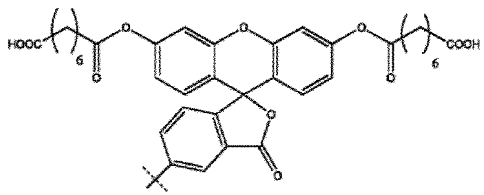 | di-(8-carboxy-hexacarbonyloxy) 5-fluorescein |
| NH | $-(CH_2CH_2O)_3CH_2CH_2NH-CH_2C(O)NH-$ | 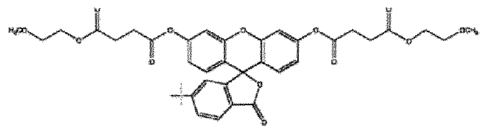 | di(methoxy-ethoxy) carbonylethyl carbonyloxy) 6-fluorescein |
| NH | $-(CH_2CH_2O)_3CH_2CH_2NH-C(S)NH-$ | 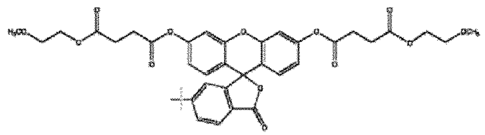 | di(methoxy-ethoxy) carbonylethyl carbonyloxy) 6-fluorescein |
| NH | $-CH_2CH_2-$ | 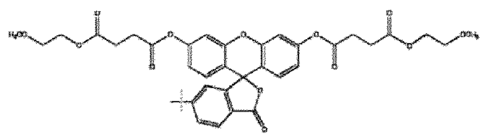 | di(methoxy-ethoxy) carbonylethyl carbonyloxy) 6-fluorescein |
| O | $-(CH_2CH_2O)CH_2CH_2O-C(S)NH-$ | 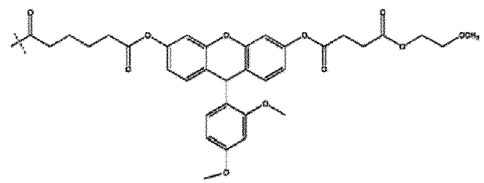 | |
| NH | $-(CH_2CH_2O)_3CH_2CH_2O-C(S)NH-$ | 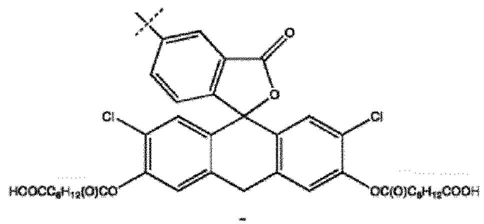 | 5',7'-dichloro-di-(8-carboxy-hexacarbonyloxy) 5-fluorescein |

or salts, tautomers and/or solvates thereof.

**3.** The conjugate of the formula:

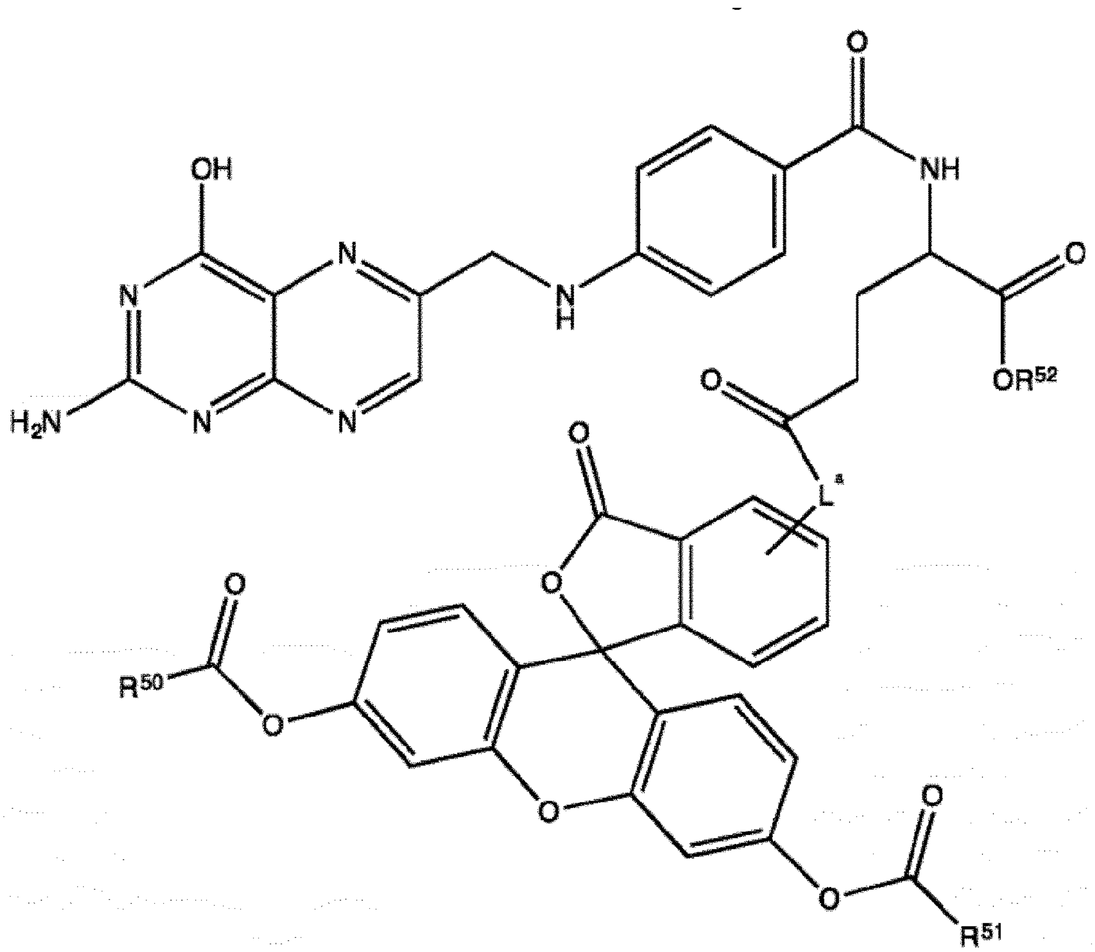

where

| $R^{50}/R^{51}$ | $R^{52}$ | $L^a$ |
|---|---|---|
| $CH_3OCH_2CH_2OC(O)CH_2CH_2C(O)O$-/ $CH_3OCH_2CH_2O$-$C(O)CH_2CH_2C(O)O$- | H | 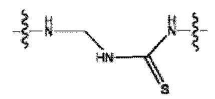 |
| $CH_3OCH_2CH_2OC(O)CH_2CH_2C(O)O$-/ $CH_3OCH_2CH_2O$-$C(O)CH_2CH_2C(O)O$- | $CH_3$ | 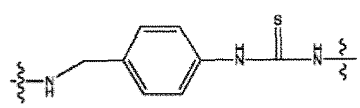 |
| $CH_3OCH_2CH_2OC(O)CH_2CH_2C(O)O$-/ $CH_3OCH_2CH_2O$-$C(O)CH_2CH_2C(O)O$- | H | -$NHCH_2H_2CH_2(OCHfH_2)_3CH_2NHC(=S)$ NH- |
| $CH_3OCH_2CH_2OC(O)CH_2CH_2C(O)O$-/ $CH_3OCH_2CH_2O$-$C(O)CH_2CH_2C(O)O$- | H | 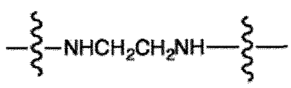 |
| $CH_3OCH_2CH_2OC(O)CH_2CH_2C(O)O$-$CH_3OCH_2CH_2O$-$C(O)CH_2CH_2C(O)O$- | H | 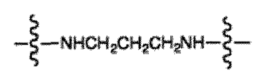 |

(continued)

| $R^{50}/R^{51}$ | $R^{52}$ | $L^a$ |
|---|---|---|
| $CH_3OCH_2CH_2OC(O)CH_2CH_2C(O)O$-/ $CH_3CH_2C(O)O$- | H | 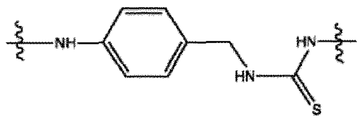 |
| $CH_3O_2C(CH_2CH_2O)3C(O)(CH_2H_2O)_3C(O)O$- $CH_3O_2C(CH_2CH_2O)_3C(O)(CH_2CH_{20})_3C(O)O$- | H | 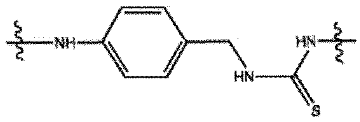 |
| $CH_3O(CH_2CH_2O)_2CH_2C(O)O$-/ $CH_{30}$ $(CH_2CH_2O)_2CH_2C(O)O$- | H | 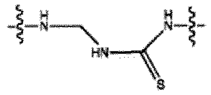 |

or salts, tautomers and/or solvates thereof.

**4.** The conjugate of the formula:

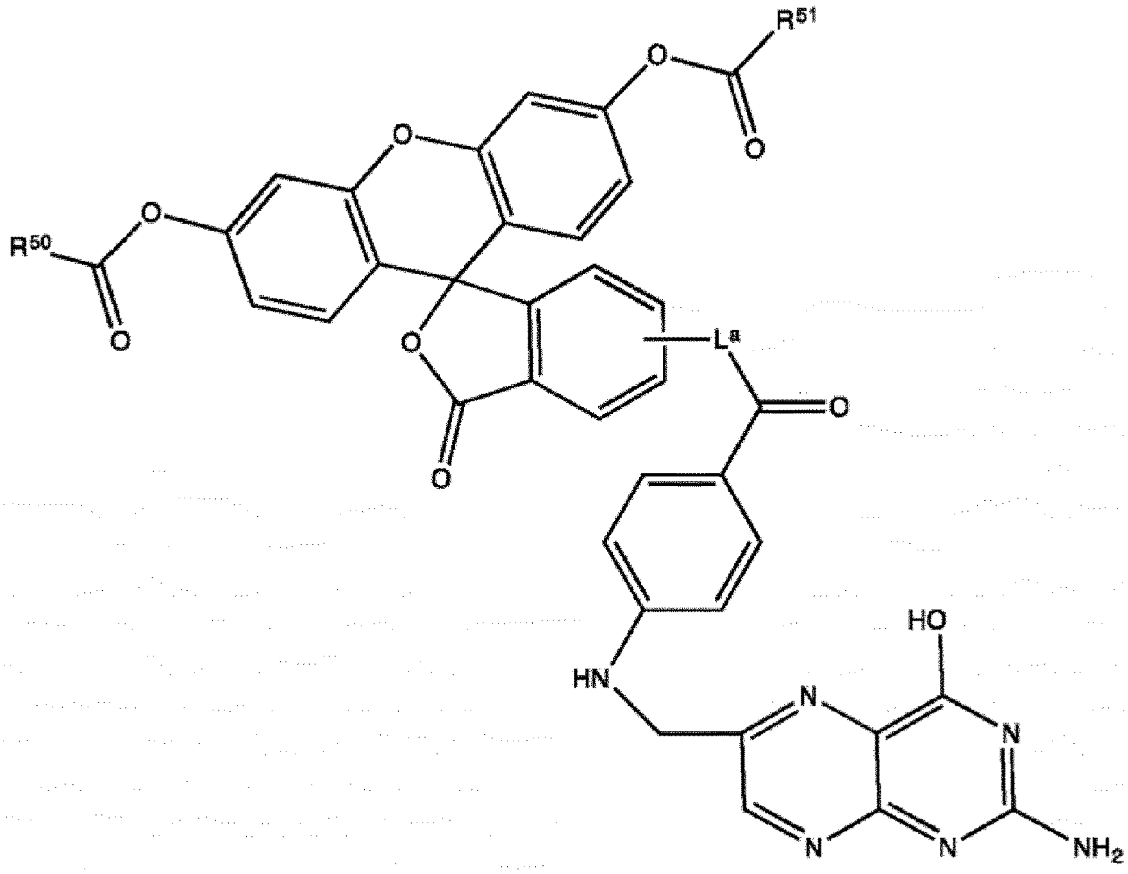

where

| $R^{50}/R^{51}$ | $L^a$ |
|---|---|
| $CH_3OCH_2CH_2OC(O)CH_2CH_2C(O)O$-/ $CH_3OCH_2CH_2OC(O)$ $CH_2CH_2C(O)O$- | 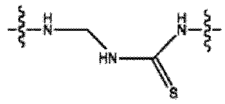 |

(continued)

| $R^{50}/R^{51}$ | $L^a$ |
|---|---|
| $CH_{3O}CH_2CH_2OC(O)CH_2CH_2C(O)O/$ $CH_3OCH_2CH_2OC(O)CH_2CH_2C(O)O-$ | 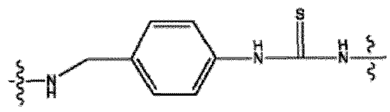 |
| $CH_3OCH_2CH_2OC(O)CH_2CH_2C(O)O-/$ $CH_3OCH_2CH_2OC(O)CH_2CH_2C(O)O-$ | $-NHCH_2CH_2CH_2(OCH_2CH_2)_3CH_2NHC(=S)NH-$ |
| $CH_3OCH_2CH_2OC(O)CH_2CH_2C(O)O-/$ $CH_3OCH_2CH_2OC(O)CH_2CH_2C(O)O-$ | 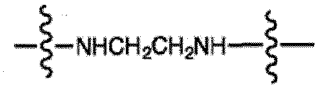 |
| $CH_3OCH_2CH_2OC(O)CH_2CH_2C(O)O-CH_3OCH_2CH_2OC(O)CH_2CH_2C[O)O-$ | 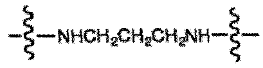 |
| $CH_3OCH_2CH_2OC(O)CH_2CH_2C(O)O-/$ $CH_3CH_2C(O)O-$ | 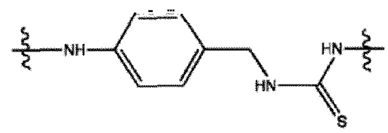 |
| $CH_3O_2C(CH_2CH_2O)_3C(O)(CH_2CH_2O)_3C(O)O-$ $CH_3O_2C(CH_2CH_2O)_3C(O)(CH_2CH_2O)_3C(O)O-$ | 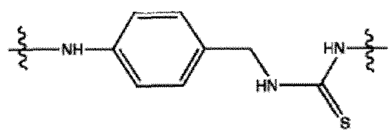 |
| $CH_3O(CH_2CH_2O)_2CH_2C(O)O-/$ $CH_3O(CH_2OH_2O)_2CH_2C(O)O-$ | 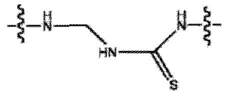 |

or salts, tautomers and/or solvates thereof.

## Patentansprüche

**1.** Konjugat der Formel:

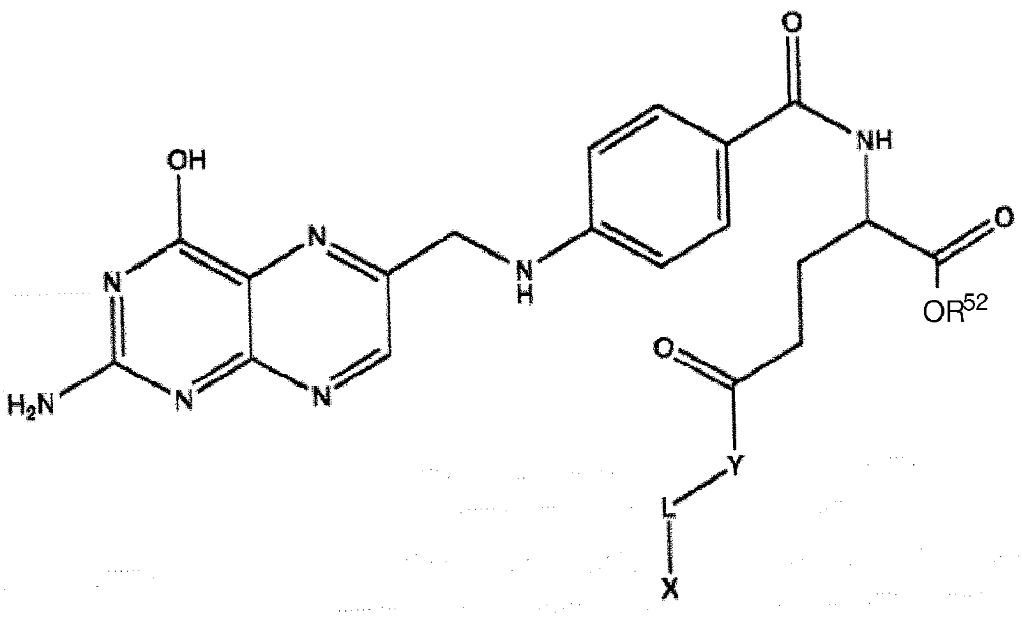

wobei $R^{52}$ für H oder $CH_3$ steht; und

| Y | L (von Y bis X) | X | Bildgebendes Mittel |
|---|---|---|---|
| NH | $-(CH_2CH_2O)CH_2CH_2NH-CH_2C(O)NH-$ | 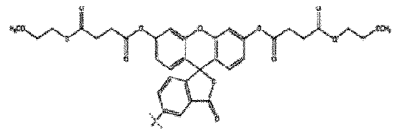 | Di(methoxyethoxy) carbonylethyl carbonyloxy) 5-fluorescein |
| NH | $-(CH_2CH_2O)_3CH_2CH_2NH-C(S)NH-$ | 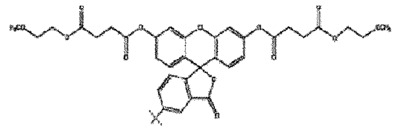 | Di(methoxy-ethoxy) carbonylethyl carbonyloxy) fluorescein |
| NH | $-CH_2CH_2-$ | 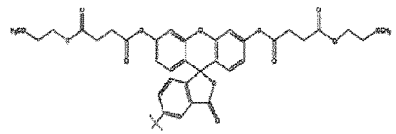 | Di(methoxy-ethoxy) carbonylethyl carbonyloxy) 5-fluorescein |
| O | $-(CH_2CH_2O)_3CH_2CH_2O-C(S)NH-$ | 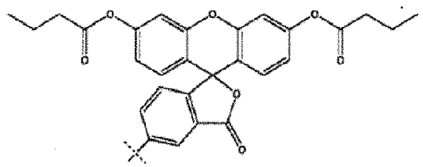 | Di-(n-butyl-carbonyloxy) 5-fluorescein |
| NH | $-(CH_2CH_2O)_3CH_2CH_2O-C(S)NH-$ | 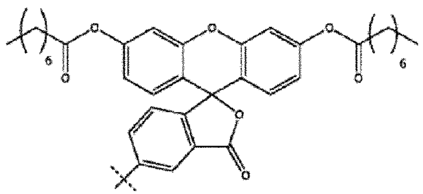 | Di-(n-hexacarbonyloxy) 5-fluorescein |
| NH | $-CH_2CH_2-$ | 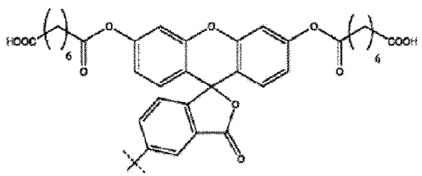 | Di-(8-carboxy-hexacarbonyloxy) 5-fluorescein |

(continued)

| Y | L (von Y bis X) | X | Bildgebendes Mittel |
|---|---|---|---|
| NH | $-(CH_2CH_2O]_3CH_2CH_2NH-CH_2C(O)NH-$ | 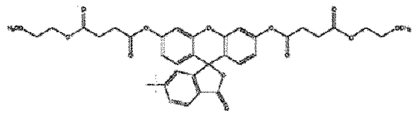 | Di(methoxy-ethoxy) carbonylethyl carbonyloxy) 6-fluorescein |
| NH | $-(CH_2CH_2O)_3CH_2CH_2NH-C(S)NH-$ | 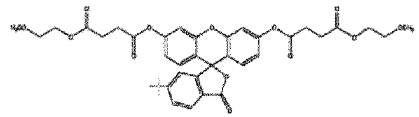 | Di(methoxyethoxy) carbonylethyl carbonyloxy) 6-fluorescein |
| NH | $-CH_2cH_2-$ | 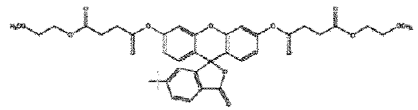 | Di(methoxyethoxy) carbonylethyl carbonyloxy) 6-fluorescein |
| O | $-(CH_2CH_2O)_3CH_2CH_2O-C(S)NH-$ | 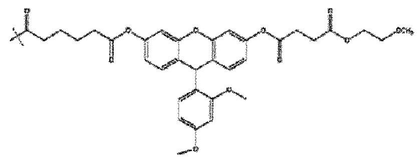 | |
| NH | $-(CH_2CH_2O)_3CH_2CH_2O-C(S)NH-$ | 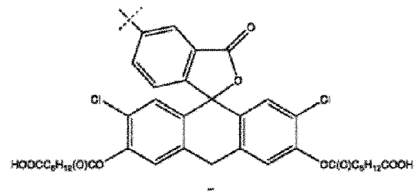 | 5',7'-Dichlor-di-(8-carboxy-hexacarbonyloxy) 5-fluorescein |
| NH | $-(CH_2)_5-$ | 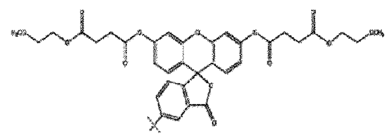 | Di(methoxy-ethoxy) carbonylethyl carbonyloxy) 5-fluorescein |

oder Salze, Tautomere und/oder Solvate davon.

**2.** Konjugat der Formel:

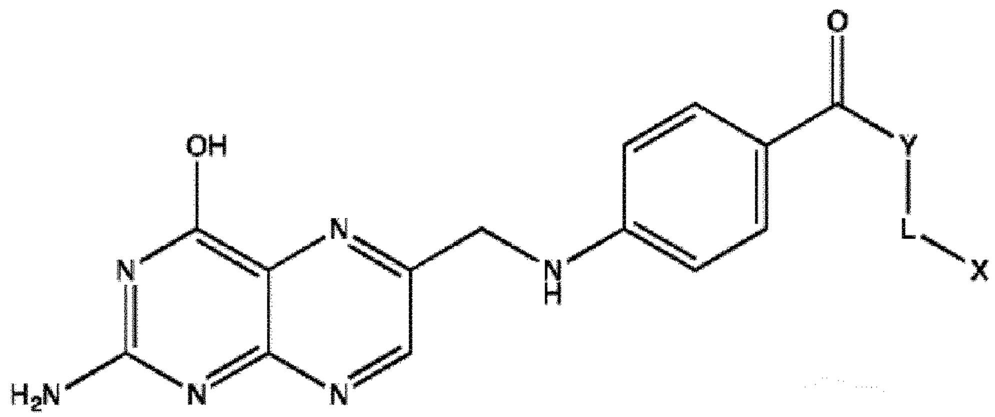

wobei

| Y | L (von Y bis X) | X | Bildgebendes Mittel |
|---|---|---|---|
| NH | $-(CH_2CH_2O)_3CH_2CH_2NH-CH_2C(O)NH-$ | 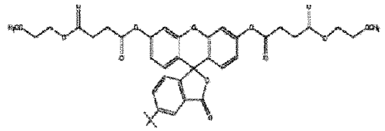 | Di(methoxy-ethoxy) carbonylethyl carbonyloxy) 5-fluorescein |
| NH | $-(CH_2CH_2O)_3CH_2CH_2NH-C(S)NH-$ | 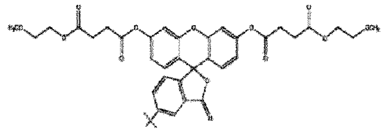 | Di(methoxy-ethoxy) carbonylethyl carbonyloxy) fluorescein |
| NH | $-CH_2CH_2-$ | 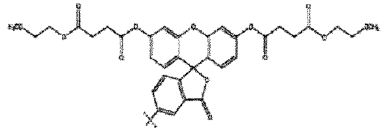 | Di(methoxyethoxy) carbonylethyl carbonyloxy) 5-fluorescein |
| O | $-(CH_2CH_2O)_3CH_2CH_2O-C(S)NH-$ | 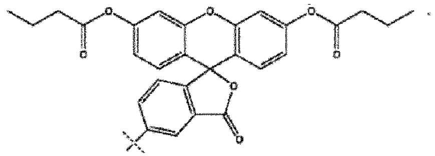 | Di-(n-butyl-carbonyloxy) 5-fluorescein |
| NH | $-(CH_2CH_2O)_3CH_2CH_2O-C(S)NH-$ | 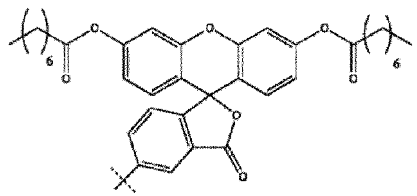 | Di-(n-hexacarbonyloxy) 5-fluorescein |
| NH | $-CH_2CH_2-$ | 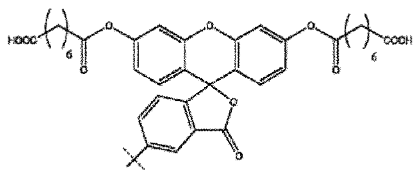 | Di-(8-carboxy-hexacarbonyloxy) 5-fluorescein |

(continued)

| Y | L (von Y bis X) | X | Bildgebendes Mittel |
|---|---|---|---|
| NH | $-(CH_2CH_2O)_3CH_2CH_2NH-CH_2C(O)NH-$ | 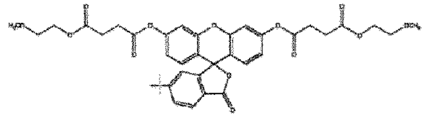 | Di(methoxy-ethoxy) carbonylethyl carbonyloxy) 6-fluorescein |
| NH | $-(CH_2CH_2O)_3CH_2CH_2NH-C(S)NH-$ | 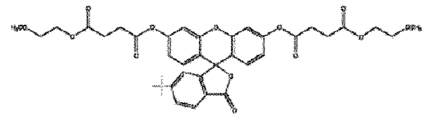 | Di(methoxy-ethoxy) carbonylethyl carbonyloxy) 6-fluorescein |
| NH | $-CH_2CH_2-$ | 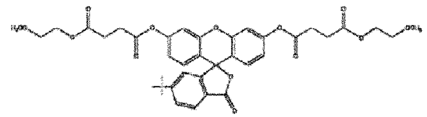 | Di(methoxy-ethoxy) carbonylethyl carbonyloxy) 6-fluorescein |
| O | $-(CH_2CH_2O)_3CH_2CH_2O-C(S)NH-$ | 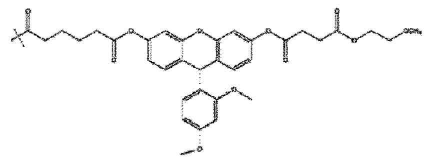 | |
| NH | $-(CH_2CH_2O)_3CH_2CH_2O-C(S)NH-$ | 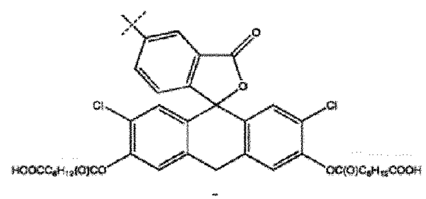 | 5',7'-Dichlor-di-(8-carboxy-hexacarbonyloxy) 5-fluorescein |

oder Salze, Tautomere und/oder Solvate davon.

**3.** Konjugat der Formel:

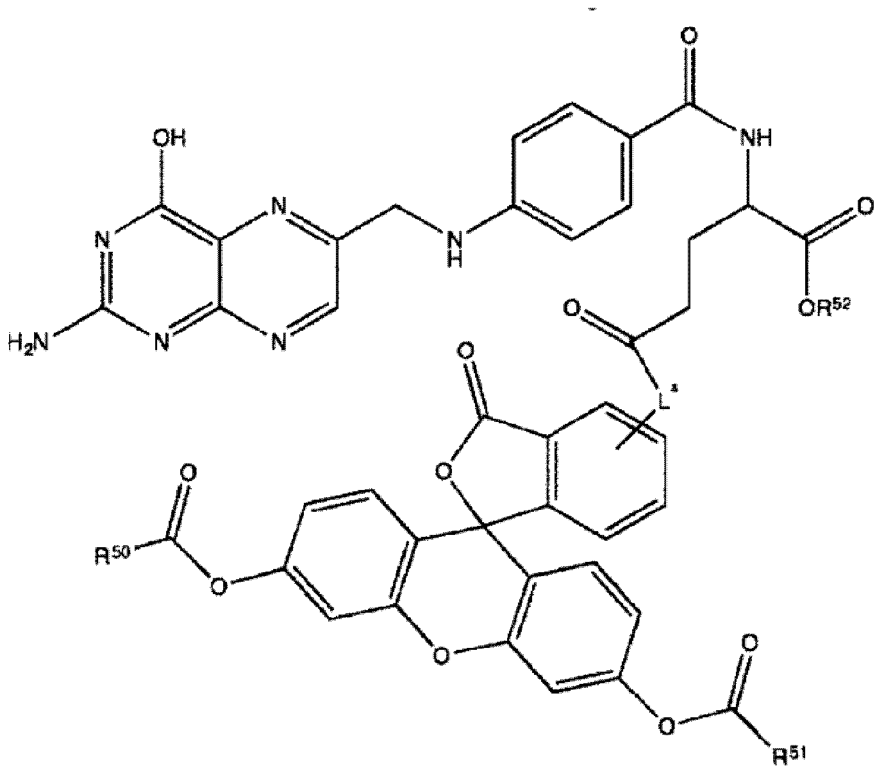

| $R^{30}/R^{51}$ | $R^{52}$ | $L^a$ |
|---|---|---|
| $CH_3OCH_2CH_2OC(O)CH_2CH_2C(O)O$-/ $CH_3OCH_2CH_2O$-$C(O)CH_2CH_2C(O)O$- | H | 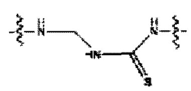 |
| $CH_3OCH_2CH_2OC(O)CH_2CH_2C(O)O$-1 $CH_3OCH_2CH_2O$-$C(O)CH_2CH_2C(0)0$- | $CH_3$ | 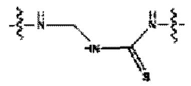 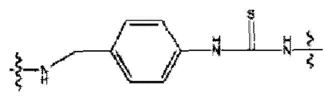 |
| $CH_3CH_2CH_2OC(O)CH_2CH_2C(O)O$-/ $CH_3OCH_2CH_2OC(O)CH_2CH_2C(O)O$- | H | $-NHCH_2CH_2(OCH_2CH_2)_3CH_2NHC(=S)NH-$ |
| $CH_3OCH_2CH_2OC(O)CH_2CH_2$ $C(O)O$-/ $CH_3OCH_2CH_2O$-$C(O)CH_2CH_2C(O)O$- | H | 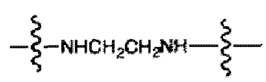 |
| $CH_3OCH_2CH_2OC(O)CH_2CH_2C(O)O$-$CH_3OCH_2CH_2OC(0)CH_2CH_2C(O)O$- | H | 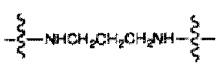 |
| $CH_3OCH_2CH_2OC(O)CH_2CH_2C(O)O$-/ $CH_3CH_2C(O)O$- | H | 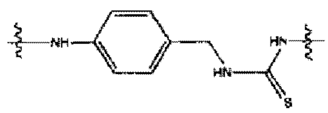 |

(continued)

| $R^{30}/R^{51}$ | $R^{52}$ | $L^a$ |
|---|---|---|
| $CH_3O_2C(CH_2CH_2O)_3C(O)(CH_2CH_2O)_3C(O)O$- $CH_3O_2C(CH_2CH_2O)_3C(O)(CH_2CH_2O)_3C(O)O$- | H | 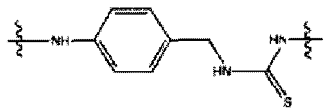 |
| $CH_3O(CH_2CH_2O)_2CH_2C(O)O$-/ $CH_3O(CH_2CH_2O)_2CH_2C(O)O$- | H | 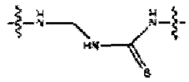 |

oder Salze, Tautomere und/oder Solvate davon.

**4.** Konjugat der Formel:

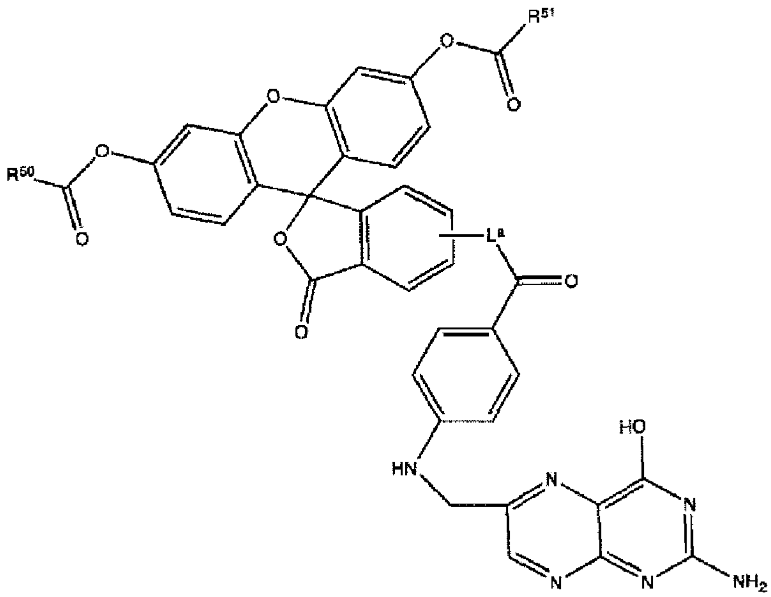

wobei wobei

| $R^{50}/R^{51}$ | $L^a$ |
|---|---|
| $CH_3OCH_2CH_2OC(O)CH_2CH_2C(O)O$-/ $CH_3OCH_2CH_2OC(O)$ $CH_2CH_2C(O)O$- | 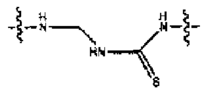 |
| $CH_3OCH_2CH_2OC(O)CH_2CH_2C(O)O$-/ $CH_3OCH_2CH_2OC(O)$ $CH_2CH_2$ $C(O)O$- | 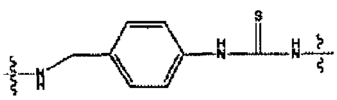 |
| $CH_3OCH_2CH_2OC(O)CH$-$_2CH_2C(O)O$-/ $CH_3OCH_2CH_2OC(O)$ $CH_2CH_2C(O)O$- | -$NCH_3CH_2CH_{2(}OCH_2CH)_2CH_2NHC(S)$ NH- |
| $CH_3OCH_2CH_2OC(O)CH_2CH_2C(O)O$-/ $CH_3OCH_2CH_2OC(O)$ $CH_2CH_2C(O)O$- | 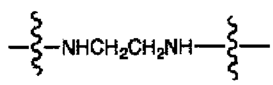 |
| $CH_3OCH_2CH_2OC(O)CH_2CH_2C(O)O$-$CH_3OCH_2CH_2OC(O)$ $CH_2CH_2C(O)O$- | 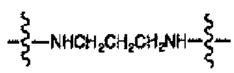 |

(continued)

| $R^{50}/R^{51}$ | $L^a$ |
|---|---|
| $CH_3OCH_2CH_2OC(O)CH_2CH_2C(O)O$-/ $CH_3CH_2C(O)O$- | 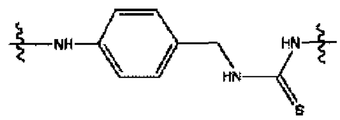 |
| $CH_3O_2C(CH_2CH_2O)_3C(O)(CH_2CH_2O)_3C(O)O$-<br>$CH_3O_2C(CH_2CH_2O)_3C(O)(CH_2CH_2O)_3C(O)O$- | 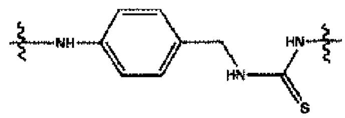 |
| $CH_3O(CH_2CH_2O)_2CH_2C(O)O$-/ $CH_3O(CH_2CH_2O)_2CH_2C(O)O$- | 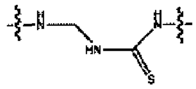 |

oder Salze, Tautomere und/oder Solvate davon.

## Revendications

**1.** Conjugué de formule :

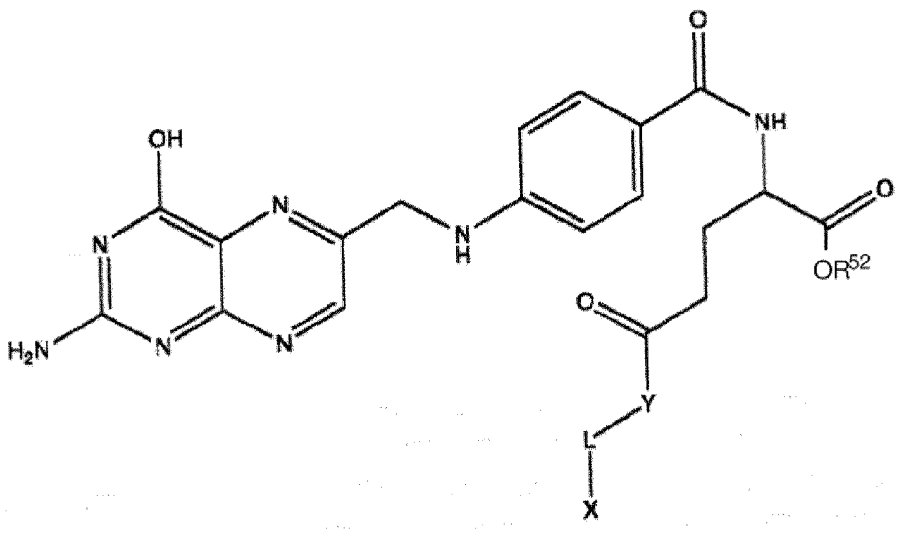

où $R^{52}$ est OH ou $CH_3$ ; et

| Y | L (de Y à X) | X | Agent d'imagerie |
|---|---|---|---|
| NH | - $(CH_2CH_2O)_3CH_2CH_2NH$-$CH_2C(O)$ NH- | 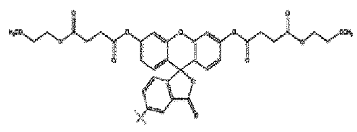 | di(méthoxy-éthoxy) carbonyléthyl carbonyloxy)-5-fluorescéine |
| NH | - $(CH_2CH_2O)_3CH_2CH_2NH$-C(S)NH- | 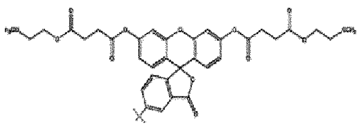 | di(méthoxy-éthoxy) carbonyléthyl carbonyloxy) fluorescéine |

(continued)

| Y | L (de Y à X) | X | Agent d'imagerie |
|---|---|---|---|
| NH | - $CH_2$-$CH_2$- | 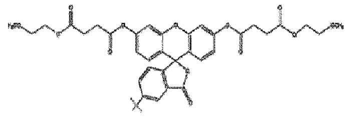 | di(méthoxy-éthoxy) carbonyléthyl carbonyloxy)-5-fluorescéine |
| O | -$(CH_2CH_2O)_3CH_2CH_2O$-C(S)NH- | 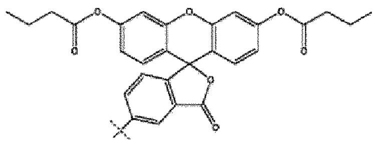 | di (*n*-butyl-carbonyloxy)-5-fluorescéine |
| NH | -$(CH_2CH_2O)_3CH_2CH_2O$-C(S) NH- | 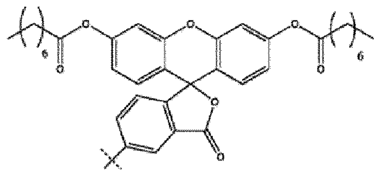 | di(n-hexacarbonyloxy)-5-fluorescéine |
| NH | -$CH_2$-$CH_2$- | 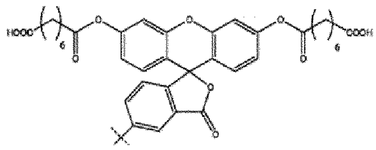 | di (8-carboxy hexacarbonyloxy) -5-fluorescéine |
| NH | -$(CH_2CH_2O)_3CH_2CH_2NH$-$CH_2C(O)$ NH- | 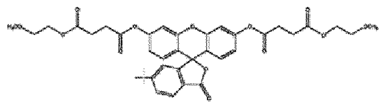 | di(méthoxy-éthoxy) carbonyléthyl carbonyloxy)-6-fluorescéine |
| NH | -$(CH_2CH_2O)_3CH_2CH_2NH$-C(S) NH- | 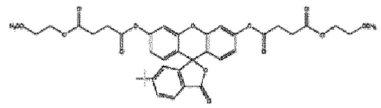 | di(méthoxy-éthoxy) carbonyléthyl carbonyloxy)-6-fluorescéine |
| NH | - $CH_2$-$CH_2$- | 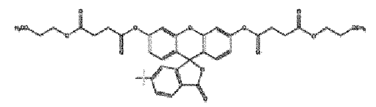 | di(méthoxy-éthoxy) carbonyléthyl carbonyloxy)-6-fluorescéine |
| O | -$(CH_2CH_2O)$ $_3CH_2CH_2O$-C(S)NH- | 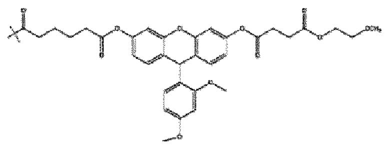 | |
| NH | -$(CH_2CH_2O)_3CH_2CH_2O$-C (S) NH- | 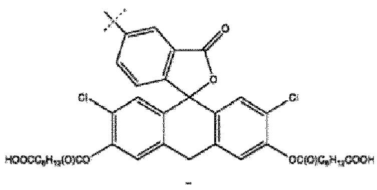 | 5',7'-dichloro-di (8-carboxy-hexacarbonyloxy) -5-fluorescéine |
| NH | - $(CH_2)_5$- | 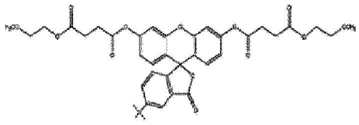 | di(méthoxy-éthoxy) carbonyléthyl carbonyloxy)-5-fluorescéine |

ou sels, tautomères et/ou solvates de celui-ci.

**2.** Conjugué de formule :

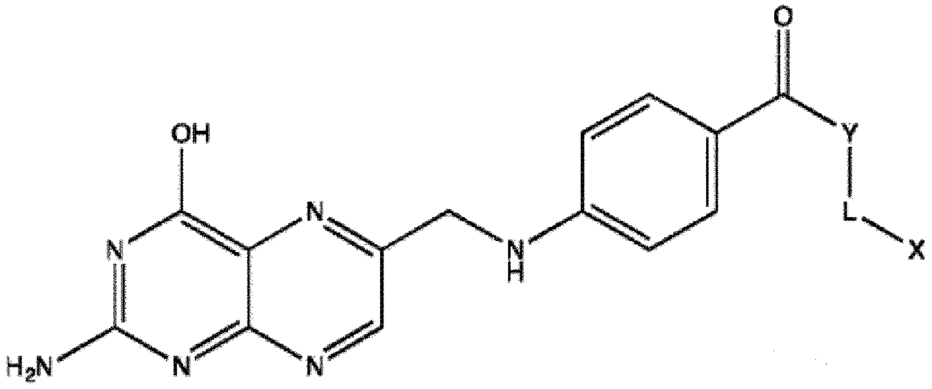

où

| Y | L (de Y à X) | X | Agent d'imagerie |
|---|---|---|---|
| NH | - $(CH_2CH_2O)_3CH_2CH_2NH-CH_2C(O)NH-$ | 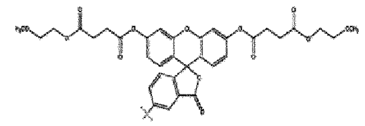 | di(méthoxy-éthoxy) carbonyléthyl carbonyloxy)-5-fluorescéine |
| NH | - $(CH_2CH_2O)\ _3CH_2CH_2NH-C(S)NH-$ | 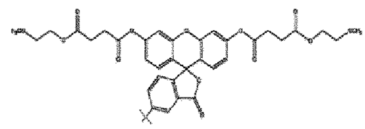 | di(méthoxy-éthoxy) carbonyléthyl carbonyloxy) fluorescéine |
| NH | $-CH_2-CH_2-$ | 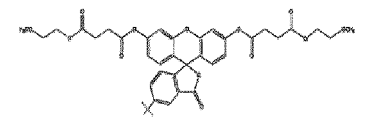 | di(méthoxy-éthoxy) carbonyléthyl carbonyloxy)-5-fluorescéine |
| O | $-(CH_2CH_2O)_3CH_2CH_2O-C(S)NH-$ | 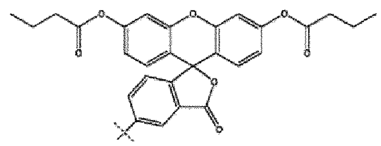 | di (n-butyl-carbonyloxy)-5-fluorescéine |
| NH | $-(CH_2CH_2O)_3CH_2CH_2O-C(S)NH-$ | 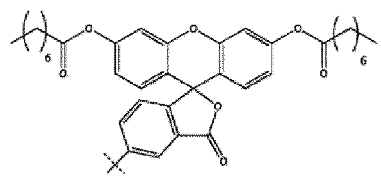 | di(n-hexacarbonyloxy)-5-fluorescéine |
| NH | $-CH_2-CH_2-$ | 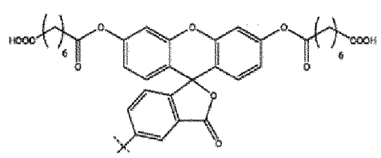 | di (8-carboxy hexacarbonyloxy) -5-fluorescéine |
| NH | $-(CH_2CH_2O)_3CH_2CH_2NH-CH_2C(O)NH-$ | 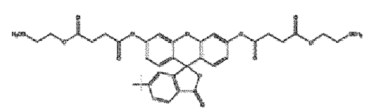 | di(méthoxy-éthoxy) carbonyléthyl carbonyloxy)-6-fluorescéine |
| NH | $-(CH_2CH_2O)_3CH_2CH_2NH-C(S)NH-$ | 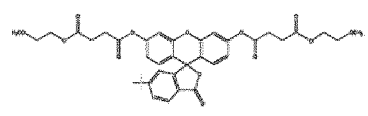 | di(méthoxy-éthoxy) carbonyléthyl carbonyloxy)-6-fluorescéine |

(continued)

| Y | L (de Y à X) | X | Agent d'imagerie |
|---|---|---|---|
| NH | $-CH_2-CH_2-$ | 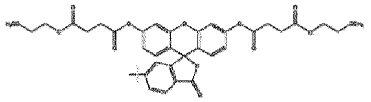 | di(méthoxy-éthoxy) carbonyléthyl carbonyloxy)-6-fluorescéine |
| O | $-(CH_2CH_2O)_3CH_2CH_2O-C(S)NH-$ | 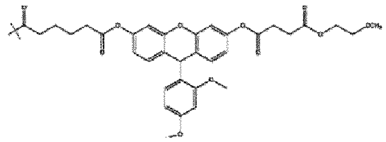 | |
| NH | $-(CH_2CH_2O)_3CH_2CH_2O-C(S)NH-$ | 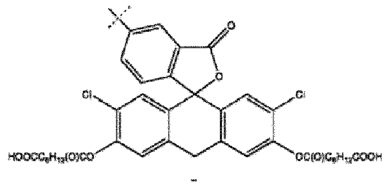 | 5',7'-dichloro-di (8-carboxy hexacarbonyloxy) -5-fluorescéine |

ou sels, tautomères et/ou solvates de celui-ci.

3. Conjugué de formule :

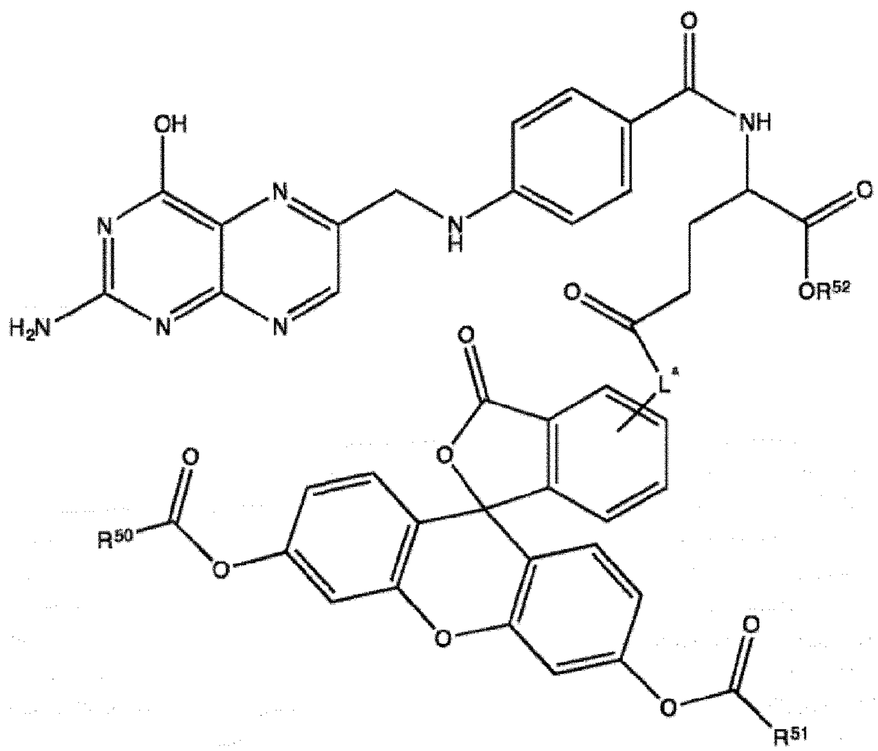

où

| $R^{50}/R^{51}$ | $R^{52}$ | $L^a$ |
|---|---|---|
| $CH_3OCH_2CH_2OC(O)CH_2CH_2C(O)O-$/ $CH_3OCH_2CH_2OC(O)CH_2CH_2C(O)O-$ | H | 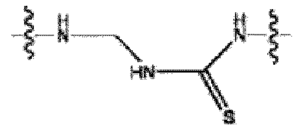 |

(continued)

| $R^{50}/R^{51}$ | $R^{52}$ | $L^a$ |
|---|---|---|
| $CH_3OCH_2CH_2OC(O)CH_2CH_2C(O)O$-/ $CH_3OCH_2CH_2OC(O)CH_2CH_2C(O)O$- | $CH_3$ | 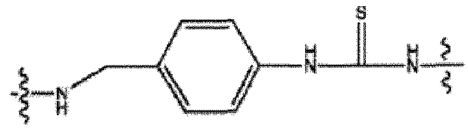 |
| $CH_3OCH_2CH_2OC(O)CH_2CH_2C(O)O$-/ $CH_3OCH_2CH_2OC(O)CH_2CH_2C(O)O$- | H | $-NHCH_2CH_2CH_2(OCH_2CH_2)_3CH_2NHC(=S)$ NH- |
| $CH_3OCH_2CH_2OC(O)CH_2CH_2C(O)O$-/ $CH_3OCH_2CH_2OC(O)CH_2CH_2C(O)O$- | H | 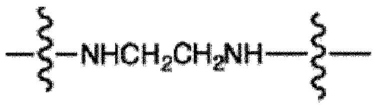 |
| $CH_3OCH_2CH_2OC(O)CH_2CH_2C(O)O$-/ $CH_3OCH_2CH_2OC(O)CH_2CH_2C(O)O$- | H | 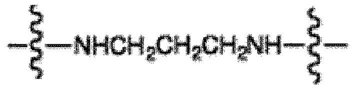 |
| $CH_3OCH_2CH_2OC(O)CH_2CH_2C(O)O$-/ $CH_3CH_2C(O)O$- | H | 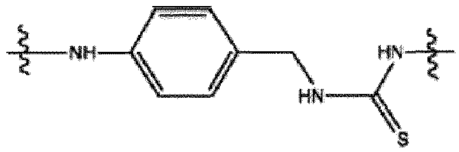 |
| $CH_3O_2C(CH_2CH_2O)_3C(O)(CH_2CH_2O)_3C(O)O$-/ $CH_3O_2C(CH_2CH_2O)_3C(O)(CH_2CH_2O)_3C(O)O$- | H | 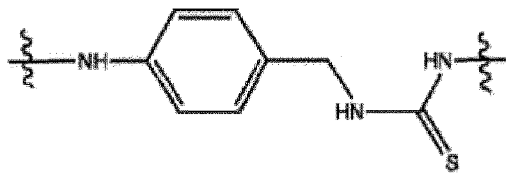 |
| $CH_3O(CH_2CH_2O)_2CH_2C(O)O$-/ $CH_3O(CH_2CH_2O)_2CH_2C(O)O$- | H | 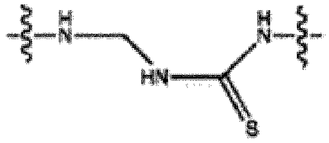 |

ou sels, tautomères et/ou solvates de celui-ci.

**4.** Conjugué de formule :

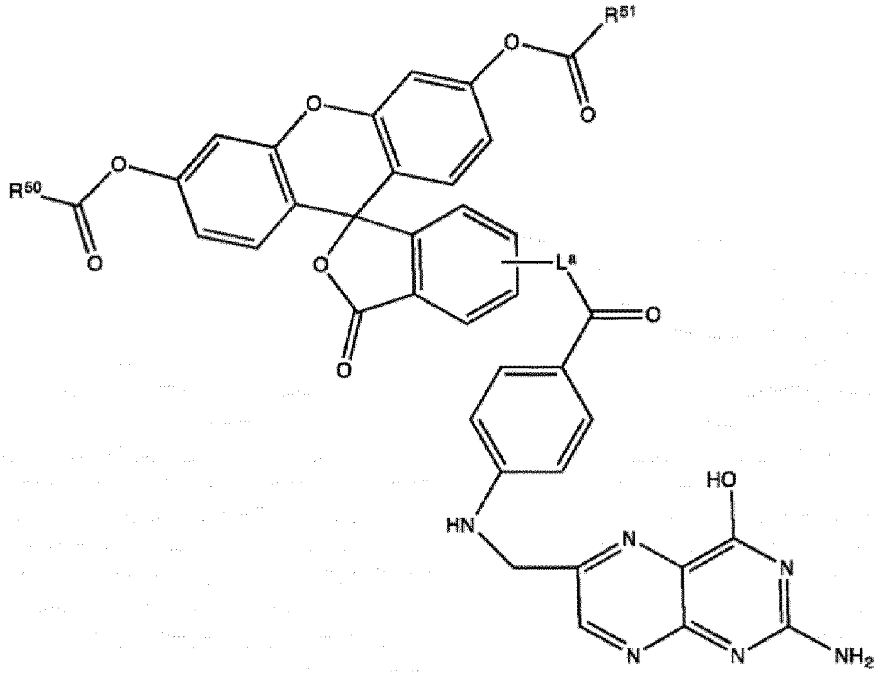

où

| $R^{50}/R^{51}$ | $L^a$ |
|---|---|
| $CH_3OCH_2CH_2OC(O)CH_2CH_2C(O)O$-/ $CH_3OCH_2CH_2O$-$C(O)CH_2CH_2C(O)O$- | 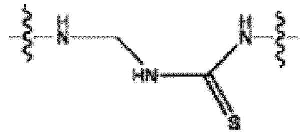 |
| $CH_3OCH_2CH_2OC(O)CH_2CH_2C(O)O$-/ $CH_3OCH_2CH_2O$-$C(O)CH_2CH_2C(O)O$- | 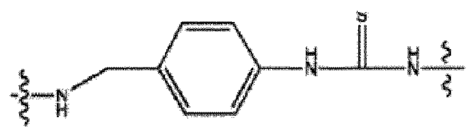 |
| $CH_3OCH_2CH_2OC(O)CH_2CH_2C(O)O$-/ $CH_3OCH_2CH_2O$-$C(O)CH_2CH_2C(O)O$- | $-NHCH_2CH_2CH_2(OCH_2CH_2)_3CH_2NHC(=S)NH-$ |
| $CH_3OCH_2CH_2OC(O)CH_2CH_2C(O)O$-/ $CH_3OCH_2CH_2O$-$C(O)CH_2CH_2C(O)O$- | 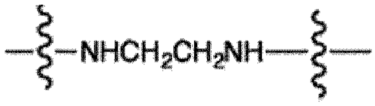 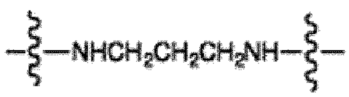 |
| $CH_3OCH_2CH_2OC(O)CH_2CH_2C(O)O$-/ $CH_3OCH_2CH_2O$-$C(O)CH_2CH_2C(O)O$- | 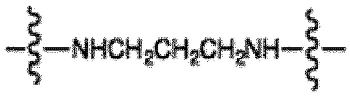 |
| $CH_3OCH_2CH_2OC(O)CH_2CH_2C(O)O$-/ $CH_3CH_2C(O)O$- | 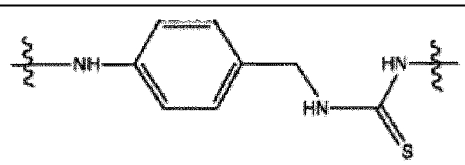 |

(continued)

| $R^{50}/R^{51}$ | $L^a$ |
|---|---|
| $CH_3O_2C(CH_2CH_2O)_3C(O)(CH_2CH_2O)_3C(O)O$-/ $CH_3O_2C(CH_2CH_2O)_3C(O)(CH_2CH_2O)_3C(O)O$- | 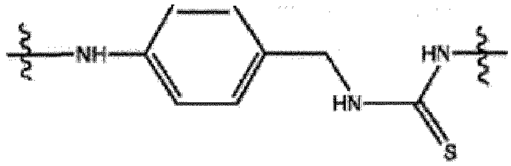 |
| $CH_3O(CH_2CH_2O)\ _2CH_2C(O)O$-/ $CH_3O(CH_2CH_2O)\ _2CH_2C(O)O$- | 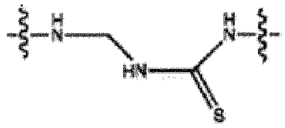 |

ou sels, tautomères et/ou solvates de celui-ci.

FIG. 1

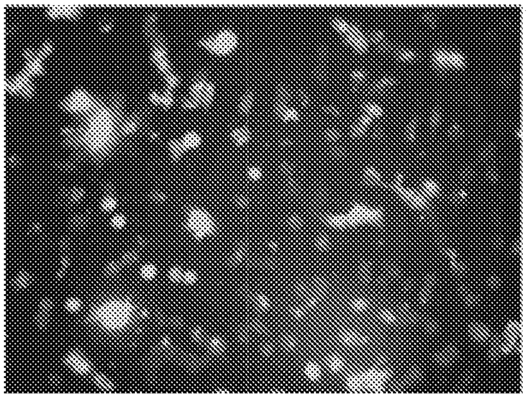

FIG. 2A and 2B

Folate competes with IS2260 for binding to folate binding protein on SKOV3 cells

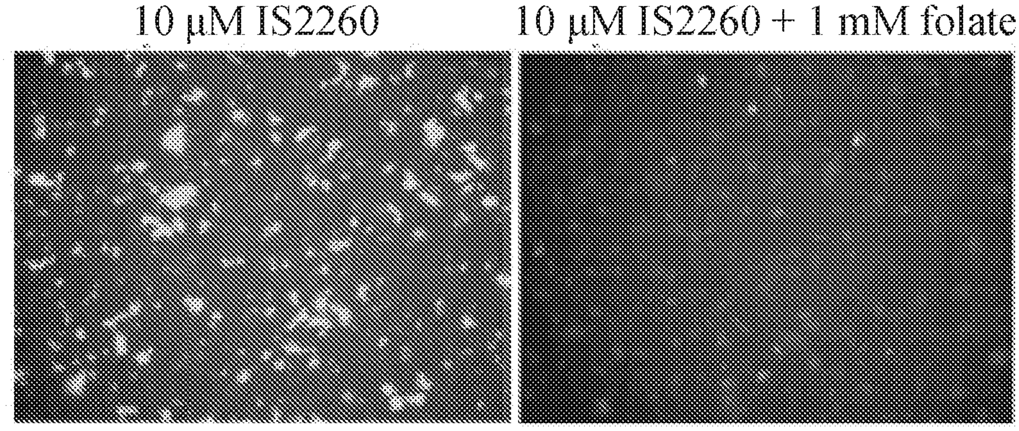

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62370130
- US 62378128 B
- US 62393524 B
- US 15595930 B
- US 15595920 B
- US 201713531 W
- US 8043603 B
- US 2013344002 A1

### Non-patent literature cited in the description


- **DHARAP et al.** *PNAS*, 2005, vol. 120 (36), 12962-12967
- **OGAWA et al.** *Mol. Pharm*, 2009, vol. 6 (2), 386-395
- **T. W. GREENE** ; **P. G. M. WUTS**. Protecting Groups in Organic Synthesis,. Wiley, 1999
- Fieser and Fieser's Reagents for Organic Synthesis. John Wiley, and Sons, 1991, vol. 1-15
- Rodd's Chemistry of Carbon Compounds. Elsevier Science Publishers, 1989, vol. 1-5
- Organic Reactions. John Wiley, and Sons, 1991, vol. 1-40
- March's Advanced Organic Chemistry,. John Wiley, and Sons, 2001
- Larock's Comprehensive Organic Transformations. VCH Publishers Inc, 1989
- **GUARAGNA et al.** *Bioconjugate Chemistry*, 2012, vol. 23, 84-96
- *J. Materials Chemistry*, 2014, vol. 2 (26), 4142-4145